# EUROPEAN PATENT APPLICATION

(11) **EP 3 511 419 A1**
(43) Date of publication of application: **17.07.2019**
(21) Application number: 17852927.7
(22) Date of filing: 13.09.2017
(51) Int. Cl.: C12P 19/04, A61K 31/739, A61K 35/74, A61P 11/00, A61P 35/00, C08B 37/00

(54) **COMPOUND OR SALT THEREOF, ANTI-INFLAMMATORY AGENT, ANTICANCER AGENT FOR LUNG CANCER, METHOD FOR PRODUCING COMPOUND OR SALT THEREOF, METHOD FOR TREATING INFLAMMATORY DISEASE, AND METHOD FOR TREATING LUNG CANCER**

(30) Priority: 23.09.2016 JP 2016186116; 08.12.2016 JP 2016238863
(71) Applicant: TFK Co., Ltd, Kobe-shi, Hyogo 652-0884 (JP)
(72) Inventor: TODA Nobuhiro, Kobe-shi Hyogo 652-0884 (JP); HIDAKA Yasuhiro, Kobe-shi Hyogo 652-0884 (JP); HAYASHI Akiyoshi, Kobe-shi Hyogo 652-0884 (JP); NISHIZAWA Takashi, Takamatsu-shi Kagawa 761-8075 (JP); NAKAMURA Shinichiro, Wako-shi Saitama 351-0198 (JP); KANIE Osamu, Wako-shi Saitama 351-0198 (JP); YAMAGUCHI Yoshiki, Wako-shi Saitama 351-0198 (JP)
(74) Representative: Isarpatent
(86) International application number: PCT/JP2017/033144
(87) International publication number: WO 2018/056150

(57) **Abstract**

The present invention provides a new compound having excellent safety which can be used as an anti-inflammatory agent, an anti-cancer agent against lung cancer, and the like. The compound or the salt thereof of the present invention is a compound represented by Formula (A) or Formula (B): 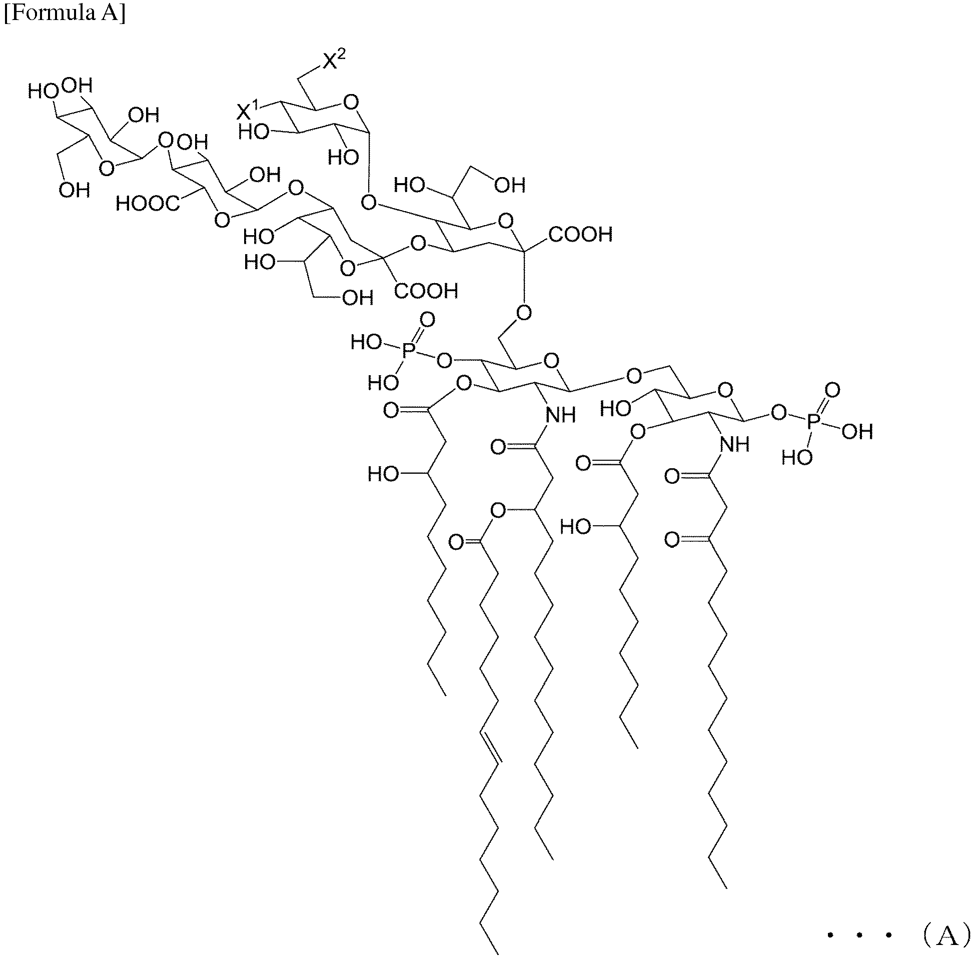 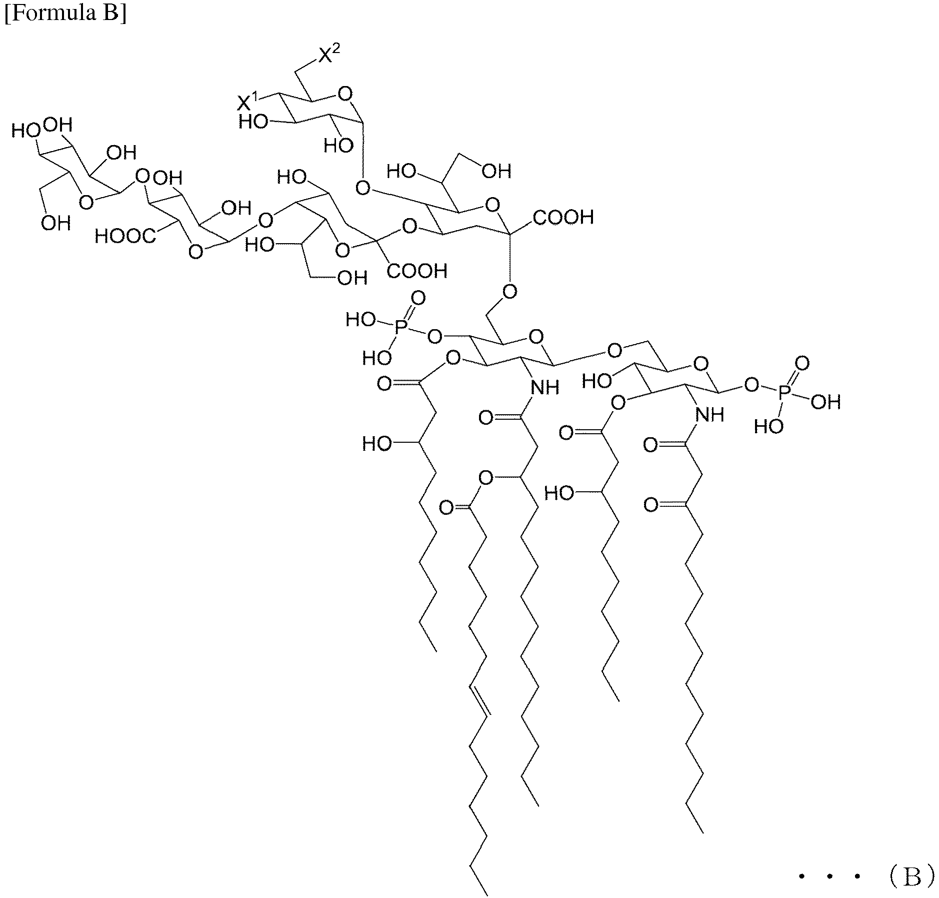 where:
X¹ is a hexose or a hydroxyl group, and
X² is a phosphate group or a hydroxyl group.

## Description

### TECHNICAL FIELD

The present invention relates to a compound or a salt thereof, an anti-inflammatory agent, an anti-cancer agent against lung cancer, a method of producing a compound or a salt thereof, a method of treating an inflammatory diseases, and a method of treating lung cancer.

### BACKGROUND ART

Various anti-inflammatory agents and anti-cancer agents against lung cancer have been proposed (for example, see Non Patent Literatures 1 and 2).

### Citation List

### Non Patent Literature

Non Patent Literature 1: Koichiro Wada, Yoshinori Kamisaki, "Anti-inflammatory effect of PPARγ agonists: basic and elinical applications," Nippon Rinsho, 2010, Vol. 68, No. 2, p.278-283
Non Patent Literature 2: Vincent T.DeVita, Jr. and Edward Chu, "A History of Cancer Chemotherapy," Cancer Res, November 1, 2008, Vol. 68, issue. 21, p.8643-8653

### SUMMARY OF INVENTION

### Technical Problem

However, there is a need for a new compound having excellent safety which can be used as an anti-inflammatory agent, an anti-cancer agent against lung cancer, and the like, and a method for producing the same.

Therefore, it is an object of the present invention to provide a new compound having excellent safety, which can be used as an anti-inflammatory agent, an anti-cancer agent against lung cancer, or the like, and a method of producing the same.

### Solution to Problem

In order to achieve the above object, the present invention provides a compound represented by Formula (A) or Formula (B) or a salt thereof: 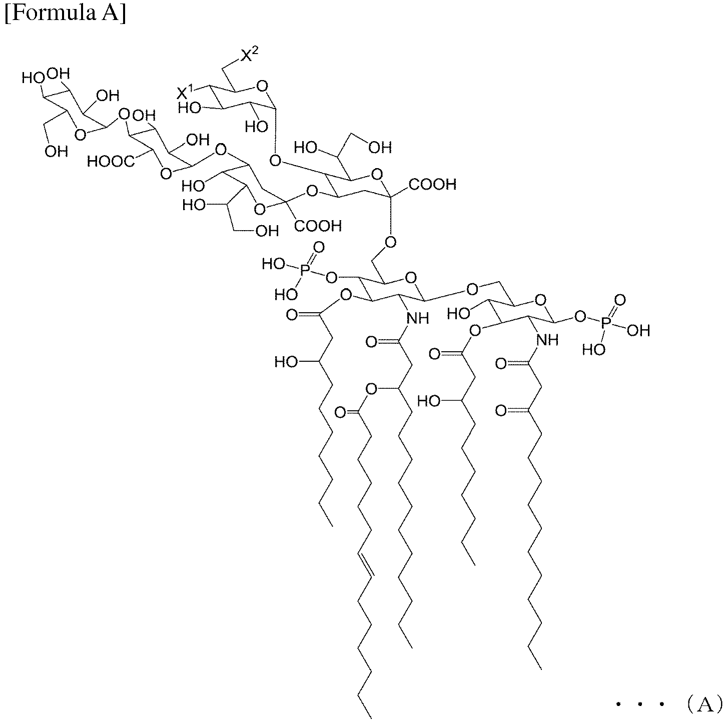 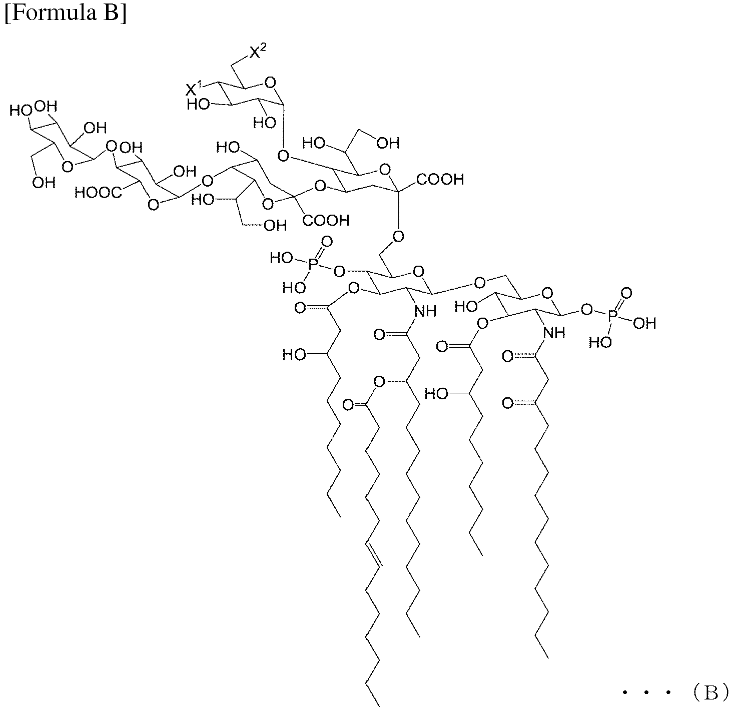 where:
X¹ is a hexose or a hydroxyl group, and
X² is a phosphate group or a hydroxyl group.

The present invention also provides a method of producing a compound or a salt thereof of the present invention, including the steps of:
extracting a crude extract including a compound represented by Formula (A) or Formula (B) or a salt thereof from at least one of Rhodobacter azotoformans BP0899 strain (Accession No.: NITE BP-644) or a culture thereof; and
isolating the compound represented by Formula (A) or Formula (B) or a salt thereof from the crude extract to purify.

### Advantageous Effects of Invention

In order to achieve the above object, the inventors of the present invention have conducted a series of studies and found that the compound represented by Formula (A) or Formula (B) or salts thereof obtained from Rhodobacter azotoformans BP0899 strain (Accession No.: NITE BP 644) or cultures thereof) has an anti-inflammatory effect and an anti-cancer effect against lung cancer. The compound represented by Formula (A) or Formula (B) or a salt thereof is highly safe and can be administered over a long period of time.

### BRIEF DESCRIPTION OF DRAWINGS

FIG. 1 is a flowchart showing an example of a crude extraction step in the production method of the present invention.
FIG. 2 is a flowchart showing an example of a purification step in the production method of the present invention.
FIG. 3 is a graph showing the IL-8 concentration of the test solution in Example 3.
FIG. 4 is a graph showing the absorbance of the test solution in Example 4.
FIG. 5 is a graph showing the absorbance of the test solution in Example 5.
FIG. 6 is a graph showing the TNF-α concentration of the test solution in Example 6.
FIG. 7 is a graph showing the TNF-α concentration of the test solution in Example 7.
FIG. 8 is a graph showing the IL-6 concentration of the test solution in Example 8-1.
FIG. 9 is a graph showing the tumor volume of mice in Example 9.
FIG. 10 is a mass spectrum obtained by subjecting the methyl esterified product to GC-MS analysis in Example 2.
FIG. 11 is a mass spectrum obtained by subjecting the hydrolyzed methyl esterified product to GC-MS analysis in Example 2.
FIG. 12 is a mass spectrum obtained by subjecting the pyrrolididized product to GC-MS analysis in Example 2.
FIG. 13 is a mass spectrum obtained by subjecting the hydrolyzed pyrrolididized product to GC-MS analysis in Example 2.
FIG. 14 is a mass spectrum obtained by subjecting the compound having the peak 4 in FIG. 12 to GC-MS analysis in Example 2.
FIG. 15 is a mass spectrum obtained by subjecting the compound having the peak D in FIG. 13 to GC-MS analysis in Example 2.
FIG. 16A is a part of a spectrum obtained by subjecting the sample solution to ¹HNMR measurements in Example 2.
FIG. 16B is part of a spectrum obtained by subjecting the sample solution to ¹HNMR measurements in Example 2.
FIG. 16C is part of a spectrum obtained by subjecting the sample solution to ¹HNMR measurements in Example 2.
FIG. 16D is a part of a spectrum obtained by subjecting the sample solution to ¹HNMR measurements in Example 2.
FIG. 17A is a part of a spectrum obtained by subjecting the sample solution to ¹³CNMR measurements in Example 2.
FIG. 17B is a part of a spectrum obtained by subjecting the sample solution to ¹³CNMR measurements in Example 2.
FIG. 17C is a part of a spectrum obtained by subjecting the sample solution to ¹³CNMR measurements in Example 2.
FIG. 17D is a part of a spectrum obtained by subjecting the sample solution to ¹³CNMR measurements in Example 2.
FIG. 18 is a spectrum of the sample solution obtained by subjecting the sample solution to ³¹PNMR measurement in Example 2.
FIG. 19 is a mass spectrum obtained by subjecting the sample solution to GC-MS analysis in Example 2.
FIG. 20 is a mass spectrum obtained by subjecting the compound having a peak of m/z = 1417.93 in FIG. 19 to GC-MS/MS analysis in Example 2.
FIG. 21 is a mass spectrum obtained by subjecting the sample solution to GC-MS analysis.
FIG. 22 is a mass spectrum obtained by subjecting the compound having a peak of m/z = 538.15 in FIG. 21 to GC-MS/MS analysis in Example 2.
FIG. 23 is a mass spectrum obtained by subjecting the compound having a peak of m/z = 474.27 in FIG. 22 to GC-MS/MS/MS analysis in Example 2.
FIG. 24 is a mass spectrum obtained by subjecting the compound having a peak of m/z = 601.17 in FIG. 22 to GC-MS/MS/MS analysis in Example 2.
FIG. 25 is a mass spectrum obtained by subjecting the compound having a peak of m/z = 557.19 in FIG. 24 to GC-MS/MS/MS analysis in Example 2.
FIG. 26 is a mass spectrum obtained by subjecting the compound having a peak of m/z = 645.12 in FIG. 24 to GC-MS/MS/MS analysis in Example 2.
FIG. 27 is a graph showing the expression levels of the PPARγ genes in Example 8-2.
FIG. 28 is a graph showing a gel filtration pattern of a sugar chain portion liberated by KOH treatment with 4 mol/L hydrazine decomposition product in Example 2.
FIG. 29 is a spectrum obtained by subjecting sample A to ¹HNMR in Example 2.
FIG. 30 is a spectrum obtained by subjecting sample A to 2D DQF-COSY measurements in Embodiment 2.
FIG. 31 is a spectrum obtained by subjecting sample A to 2D NOESY measurements in Example 2.
FIG. 32 is a spectrum obtained by subjecting sample A to 2D ¹H-³¹P HMBC measurements in Example 2.
FIG. 33 is a schematic diagram of sample A in Example 2.

### DESCRIPTION OF EMBODIMENTS

In the production method of the present invention, the extraction process in the crude extraction step may be an extraction process using an organic solvent that insolubilizes a protein. In this case, the extraction solvent may be phenol.

In the manufacturing process, in the crude extraction step, at least one of Rhodobacter azotoformans BP0899 strain (Accession No.: NITE BP 644) and a culture thereof may be subjected a decolorization process of a pigment prior to the extraction process. In this case, the decolorization process of the pigment may be a decolorization process using at least one selected from the group consisting of acetone, methanol and chloroform.

In the production method of the present invention, the crude extract may be subjected to a filtration process in the crude extraction step.

In the production method of the present invention, in the purification step, the crude extract may be subjected to an enzyme treatment process and an extraction process using an organic solvent that insolubilizes a protein. In this case, the enzyme treatment process may be enzyme treatment process using at least one of a nuclease and a protease, and the organic solvent may be phenol.

In the production method of the present invention, in the purification step, an extract after the extraction process may be subjected to a filtration process.

The present invention is described below in detail.

### <New compound>

As described above, the compound or the salt thereof of the present invention is a compound represented by Formula (A) or Formula (B) or a salt thereof. In Formulae (A) and (B), X¹ and X² have four kinds of combinations such as a hexose and phosphate group, a hydroxyl and a hydroxyl group, a hexose and a hydroxyl group, a hydroxyl and a phosphate group. Among them, the combinations of a hexose and a phosphate group and a hydroxyl group and a hydroxyl group are preferable. When X¹ is a hexose, for example, the hydroxyl group on the 2-carbon is bonded to the 4-carbon of the hexose to which X¹ is to be bonded. The compound or the salt thereof of the present invention is a compound represented by Formula (A1) or Formula (B1) or a salt thereof when X¹ and X² are a hexose and a phosphate group, respectively, in Formula (A) or Formula (B), and is a compound represented by Formula (A2) or Formula (B2) or a salt thereof when X¹ and X² are both hydroxyl groups in Formula (A) or Formula (B). The compound or the salt thereof of the present invention is hereinafter also referred to as "novel compound of the present invention". The novel compound of the present invention can be obtained, for example, by the production method described below. However, the production method described below is merely an example, and the present invention is not limited thereto.

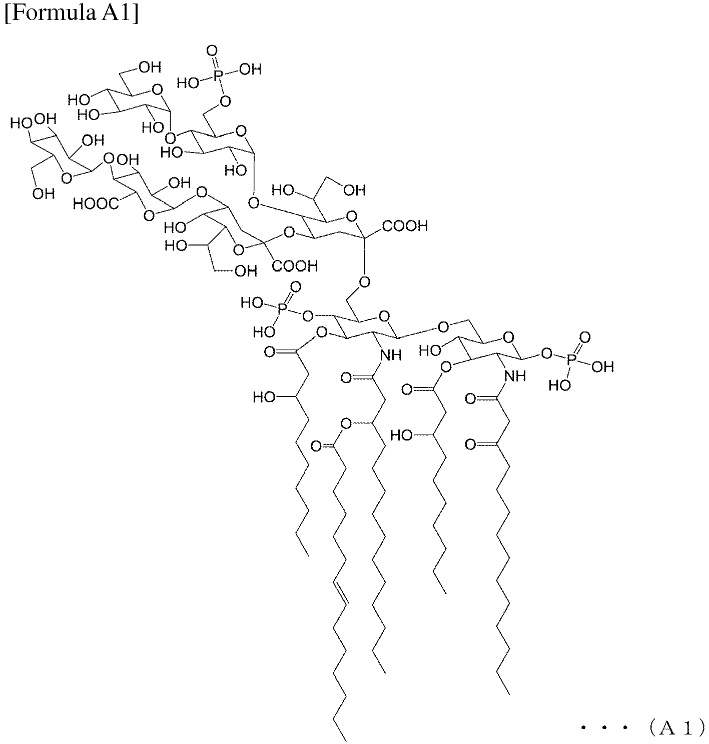

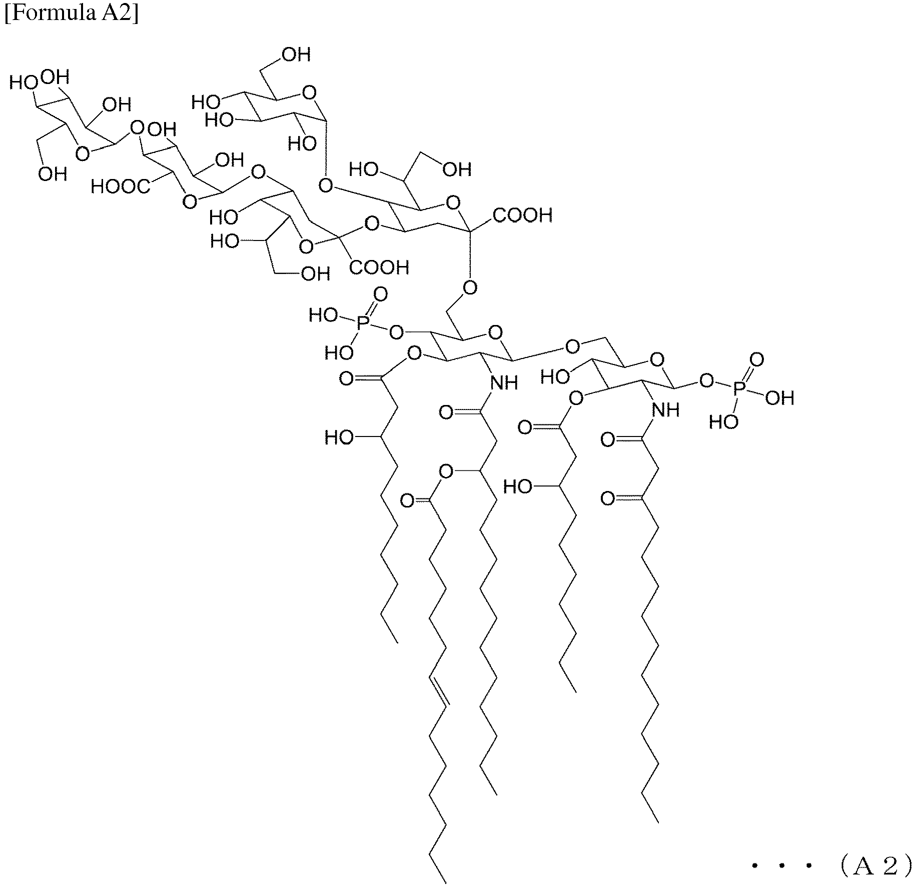

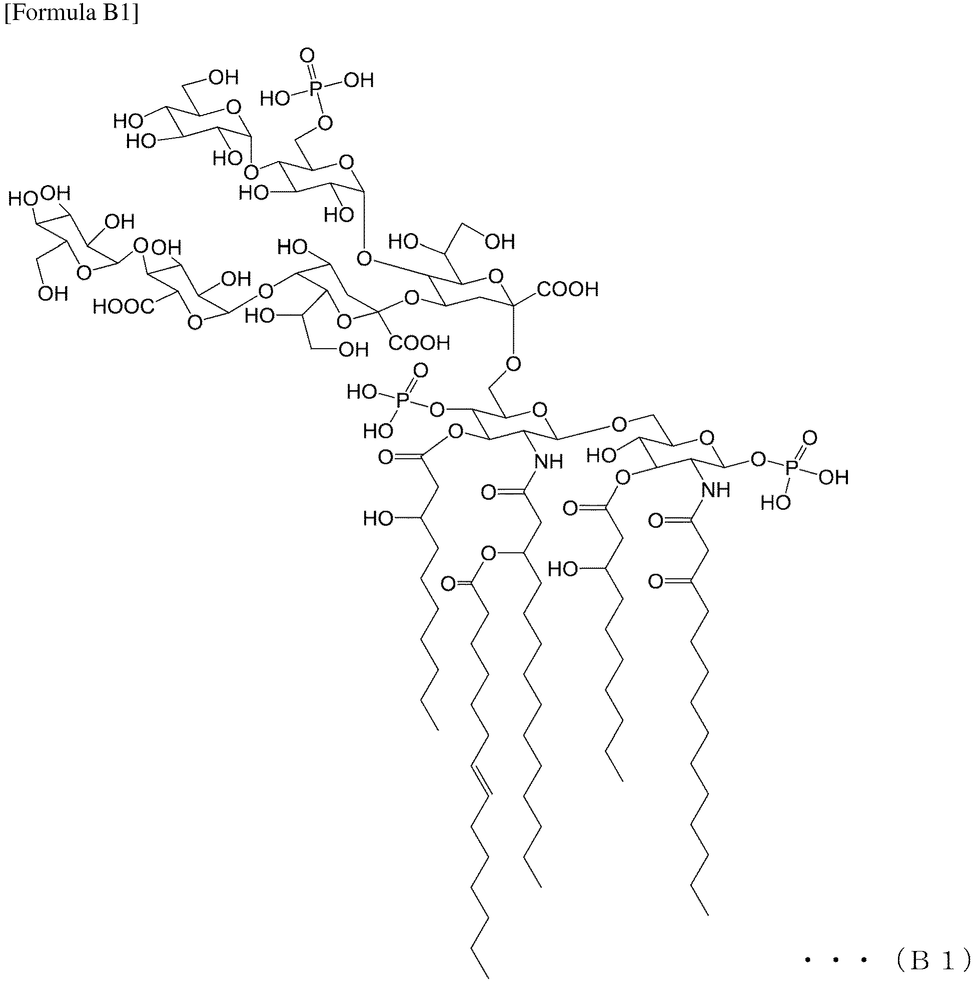

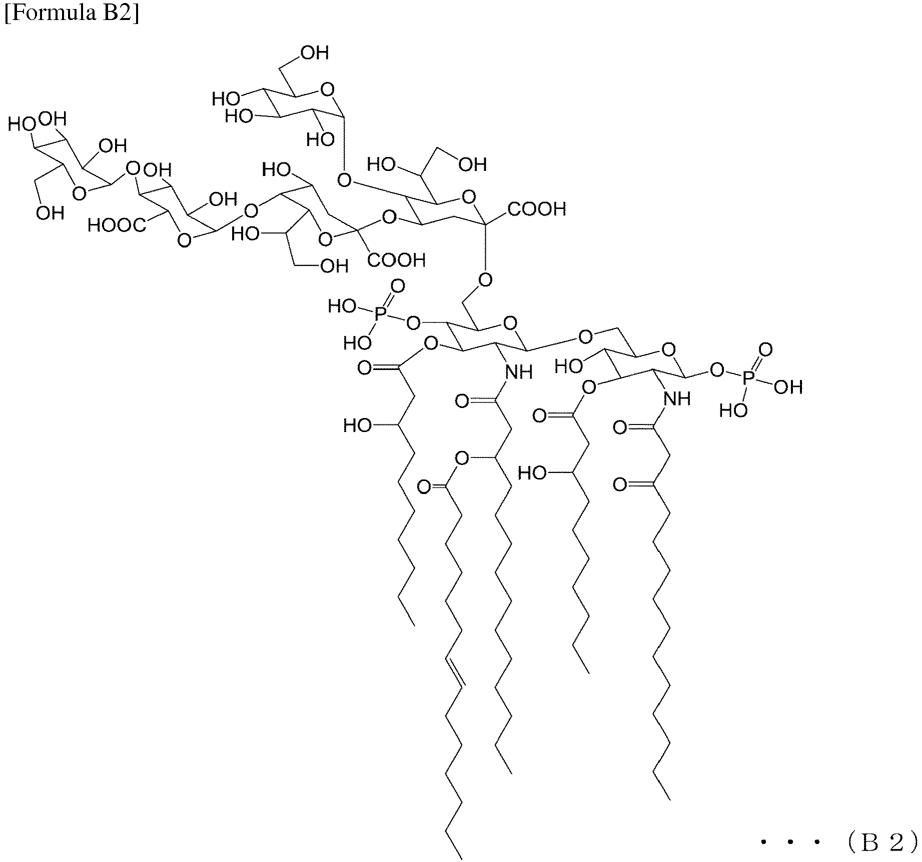

In the novel compound of the present invention, the hexose may be, for example, glucose.

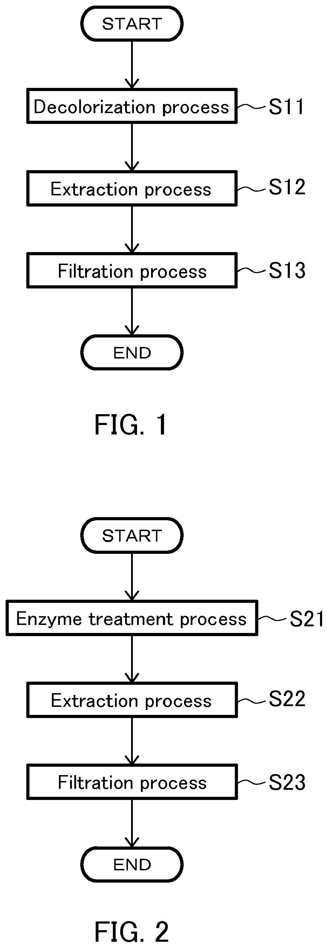

The novel compound of the present invention may be used in any application, and can be used as, for example, an anti-inflammatory agent described below, a material of an anti-cancer agent against lung cancer, and the like. The novel compounds have the function of inhibiting the production of inflammatory cytokines such as NF-KB (Nuclear Factor-kappa B), TNF-α (Tumor Necrosis Factor α), and IL-6 (Interleukin 6), as demonstrated in the Examples below. The novel compounds also function to activate TLR4 (Toll-like receptor 4), a type of Toll like receptor, as demonstrated in the Examples below. Activation of TLR4 is known to stimulate the production of type I interferons with anti-inflammatory effects (Nina Maeshima and Rachel C.Fernandez, "Recognition of lipid A variants by the TLR4-MD-2 receptor complex", Frontiers in Cellular and Infection Microbiology, February 2013, volume3, Article3, p. 2, FIGURE 2). Thus, the novel compounds of the present invention have an anti-inflammatory effect. In addition, the novel compounds of the present invention also have the function of suppressing the growth of lung cancer, as demonstrated in the Examples below.

### <Anti-inflammatory Agents and Methods of Treating Inflammatory Diseases>

The anti-inflammatory agent of the present invention suppresses inflammation by the anti-inflammatory effect of the compound or the salt thereof of the present invention, and is not limited in any way except for containing the novel compound of the present invention. The diseases in which inflammation can be suppressed by the anti-inflammatory agent of the present invention include, for example, inflammatory bowel diseases such as ulcerative colitis and Crohn's disease, inflammatory skin diseases such as psoriasis and dermatitis, encephalitis, hepatitis, nephritis, pneumonia, bronchitis, vasculitis, meningitis, thyroiditis, diabetes mellitus, inflammatory bile disease, and cancer accompanied by inflammation, and are not particularly limited. According to the anti-inflammatory agent of the present invention, the anti-inflammatory effect of the novel compound of the present invention also exerts the analgesic effect of pain associated with inflammation.

Such anti-inflammatory dosage forms include, but are not limited to, powders, fine granules, tablets, coated tablets, capsules, troches, solutions, and the like. The composition of the anti-inflammatory agent is not particularly limited, and may contain, for example, various additives such as excipients, binders, lubricants, disintegrants, absorption accelerators, emulsifiers, stabilizers, preservatives, and the like in addition to the novel compound of the present invention. The anti-inflammatory agent can be produced by a commonly used formulation technique or the like.

A method of treating an inflammatory disease of the present invention includes the step of administering an anti-inflammatory agent of the present invention including the novel compound. In the present invention, "treatment" includes, for example, amelioration (improvement) of a symptom, resolution (complete cure) of a symptom, prevention of worsening of a symptom, prevention, and the like, and the same applies to a method of treating lung cancer described below. The animal species to which the anti-inflammatory agent is administered is not particularly limited, and includes, for example, humans, non-human mammals such as monkeys, cows, pigs, dogs, cats, birds such as chickens, fish and shellfish, and the like. The method of administration is not particularly limited and includes, for example, oral administration or parenteral administration, and the parenteral administration includes, for example, transdermal absorption, injection, suppository administration, and the like. The dosage of the anti-inflammatory agent can be appropriately set according to, for example, the animal species, the age, and the like, and is not particularly limited.

### <Anticancer-Agents against Lung Cancer and Methods of Lung Cancer Treatment>

The anti-cancer agent against lung cancer of the present invention suppresses the proliferation of lung cancer by the anti-cancer effect of the compound or the salt thereof of the present invention (the novel compound), and is not limited in any way except for containing the novel compound of the present invention. The method of treating lung cancer of the present invention also includes the step of administering an anti-cancer agent against lung cancer of the present invention including the novel compound. The dosage form of the anti-cancer agent, the animal species to which the anti-cancer agent is administered, and the method of administering the anti-cancer agent are the same as the dosage form of the anti-inflammatory agent, the animal species to which the anti-inflammatory agent is administered, and the method of administering the anti-inflammatory agent.

### <Method of Preparing Novel Compounds>

As described above, the method of producing a compound represented by Formula (A) or Formula (B) or a salt thereof of the present invention includes the steps of: extracting a crude extract including a compound represented by Formula (A) or Formula (B) or a salt thereof from at least one of Rhodobacter azotoformans BP0899 strain (Accession No.: NITE BP-644) and a culture thereof; and isolating the compound represented by Formula (A) or Formula (B) or a salt thereof from the crude extract to purify:

### [Crude extraction process]

As described above, the crude extraction step is a step of extracting a crude extract including a compound of Formula (A) or Formula (B) or a salt thereof from at least one of Rhodobacter azotoformans BP0899 strain NITE BP 644) and a culture thereof.

First, at least one of the aforementioned Rhodobacter azotoformans BP0899 strain (Accession No. NITE BP-644) and a culture thereof will be described. It is preferable that at least one of the Rhodobacter azotoformans BP0899 strain (Accession No. NITE BP-644) and the culture thereof has the following mycological characteristics (1) to (30). The above-mentioned BP0899 strains were deposited with the Deposit Center for Patents and Organisms of the National Institute for Product Evaluation and Technology (2-5-8 Kazusa, Kisarazu-shi, Chiba, Japan) under the Accession No. NITE P-644 (deposit date: September 12, 2008) and internationally deposited under the Accession No. NITE BP-644 (transfer date: October 27, 2010).
(1) Cell shape: Rod shape or oval shape
(2) Polymorphism: None
(3) Cell size: 0.8 µm ×1.0 µm
(4) Motility: Yes
(5) Spores: None
(6) Glossy in common agar cultures: Yes
(7) Chromogenesis in common agar cultures: Yes
(8) Surface growth in normal broth cultures: None
(9) Turbidity of the medium in normal broth cultures: Yes
(10) Gelatin liquefaction in gelatin puncture cultures: Negative
(11) Coagulation in litmus milk cultures: None
(12) Liquefaction in litmus milk cultures: None
(13) Gram stain: Negative
(14) Nitrate reduction: None
(15) Denitrification reaction: None or Yes
(16) MR test: Negative
(17) Indole production: None
(18) Hydrogen sulfide production: None
(19) Starch Hydrolysis: None
(20) Use of citric acid (Christensen): None
(21) Use of inorganic nitrogen source (ammonium salt): Yes
(22) Catalase production: positive
(23) Oxidase production: positive
(24) Anaerobic viability: Yes
(25) O-F test (oxidation/fermentation): negative/negative
(26) β-galactosidase activity: negative
(27) Arginine dihydrolase activity: negative
(28) Lysine decarboxylase activity: negative
(29) Tryptophan deaminase activity: negative
(30) Gelatinase activity: negative
The base sequence of the 16S rRNA of the BP0899 strain is preferably the base sequence represented by SEQ ID NO: 1.
At least one of the BP0899 strain and the culture thereof may further exhibit the properties shown in the table (31) below, for example, under aerobic culture conditions in the dark. In the table (31), "-" indicates no production, and "+" indicates production.
(31) Acid and gas production from sugars

| Substrate | acid production/gas production |
|---|---|
| L-arabinose | -/- |
| D-Glucose | -/- |
| D-fructose | -/- |
| Maltose | -/- |
| Lactose | -/- |
| D-sorbitol | -/- |
| Inositol | -/- |
| D-xylose | -/- |
| D-mannose | -/- |
| D-galactose | -/- |
| Saccharose | -/- |
| Trehalose | -/- |
| Glycerin | -/- |

The mycological characteristics may be evaluated, for example, from the results of a main cultivation conducted after a pre-cultivation. The pre-cultivation may be performed, for example, by inoculating the BP0899 strain on a nutrient agar medium and culturing the agar medium at 30°C for 24 hours. The conditions of the main cultivation can be appropriately set according to the evaluation method of each mycological characteristic. Specifically, the culturing conditions (1) to (5) are, for example, aerobic culturing at 30°C and in the dark using nutrient agar medium, and the culturing conditions (6) to (7) are, for example, anaerobic culturing at 30°C and in the light using nutrient broth medium, and the culturing conditions (8) to (12) are, for example, aerobic culturing at 30°C and in the dark using each medium, and (13), (14), (16), (17), (19) to (23), oxidation test of (25), (26), (29), (30), and (31) are, for example, aerobic culturing in the dark, and (15), (18), (24), fermentation test of (25), (27), and (28) are, for example, anaerobic culturing in the dark. The test method of these mycological characteristics is not particularly limited, and a conventionally known method can be employed. Specifically, for example, the methods described in "Cowan and Steel's Manual for the Identification of Medical Bacteria." (U.K.), Barrow G. I. and Feltham R. K. A., 3rd edition, Cambridge University Press, 1993; "SHIN SAIKIN BAICHIGAKU KOZA Volume 2" (Tokyo) Sakazaki et al., 2nd edition, Kinki University Publishing, 1988; "BISEIBUTSU NO BUNRUI TO DOTEI (Volume 2)" (Tokyo), edited by Hasegawa, Society Publishing Center, 1985; **"**SHINPEN DOJO BISEIBUTSUGAKU JIKKEN" (Tokyo), edited by Soil Microbial Study Society, Yokendo, 1992, and the like can be cited. As the test method (15), for example, the method of Komagata et al. described in the above-mentioned "BISEIBUTSU NO BUNRUI TO DOTEI (Volume 2)", the method described in the above-mentioned "SHINPEN DOJO BISEIBUTSUGAKU JIKKEN" using Giltay medium, the sewage method using PYN medium, and the like can be employed. In the method of Komagata et al., the result with growth and gas formation under anaerobic culture conditions using 1% sodium nitrate broth are determined to be positive for denitrification reaction. In the method using the Giltay medium, the result with the gas-evolving and dark blue color produced under anaerobic culture conditions using the Giltay medium (pH 7.0 to pH 7.2) in a Durham tube is determined to be positive for denitrification. The Giltay medium is a medium containing Solution A (1 g of KNO₃, 1 g of asparagine, 5 mL of 1% bromothymol blue-alcohol solution and 500 mL of distilled water) and Solution B (8.5 g of sodium citrate, 1 g of MgSO₄·7H₂O, 0.05 g of FeCl₃·6H₂O, 1 g of KH₂PO₄, 0.2 g of CaCl₂·6H₂O and 500 mL of distilled water). In addition, for example, a commercially available bacterial identification kit may be used for the test method. The kit is not particularly limited, and for example, a bacterial identification kit API20E manufactured by Biomeleu Corporation or the like can be used.
At least one of the BP0899 strain and the culture thereof may have, for example, the following mycological characteristics (32) to (40).
(32) Colony color: Red
(33) Gelatin puncture cultures: No growth
(34) VP test: Negative
(35) Use of citric acid (Koser): Yes
(36) Use of inorganic nitrogen source (nitrate): Yes
(37) Urease activity: negative
(38) Growing pH range: 5 to9
(39) Acid production from D-mannitol: produced
(40) Gas Production from D-Mannitol: No Production

The test method of the mycological characteristics of the above-mentioned (32) to (40) is not particularly limited, and a conventionally known method can be adopted. Specifically, for example, a method described in the aforementioned documents or the like can be cited. In addition, for example, a commercially available bacterial identification kit may be used for the test method. The kit is not particularly limited, and for example, the aforementioned bacterial identification kit or the like can be used.

The source of the BP0899 strain is not particularly limited, and for example, soils, sea water, river water, lake water, marsh water, and the like can be cited. Examples of the soil include land, sea bed, river bed, lake bottom, and marsh bottom soil, sand, mud, and the like, and are not particularly limited.

As a method of isolating the BP0899 strain, for example, a conventionally known collection method, culture method, or the like can be used, and there is no particular limitation. As the isolation method, for example, when the collection source is lake water, the collected lake water may be filtered by a filter or the like, and the filtrate may be cultured on agar medium or the like to isolate the BP0899 strain from the obtained colonies. For example, when the collection source is mud, the collected mud may be suspended in a buffer solution or the like, and then the suspension may be centrifuged, and the obtained supernatant may be cultured on agar medium or the like to isolate the BP0899 strain from the obtained colonies. The isolated BP0899 strain may further be cultured, for example, in a fluid medium.

In the culture of the BP0899 strain, the culture medium is not particularly limited, and for example, a medium containing a lower fatty acid, a medium containing a malic acid, a Media for revival of L-dried specimens 802 "DAIGO" (manufactured by NIHON PHARMACEUTICAL CO., LTD), a MYS medium (Hiraishi and Kitakawa, Bulletin of the Japanese Society of Scientific Fisheries, 1984, Vol. 50, No. 11, p. 1929-1937), a modified MYS medium, a growth medium, and the like are listed, and preferably, a medium containing a low fatty acid, a medium containing a malic acid, and a Media for revival of L-dried specimens 802 "DAIGO" (manufactured by NIHON PHARMACEUTICAL CO., LTD).

The medium containing lower fatty acids and the medium containing malic acid include, for example, a medium obtained by adding biotin, vitamin B₁, nicotinic acid, lower fatty acids or sodium salts of malic acid to the basal medium shown in Table 1 below. The lower fatty acid is not particularly limited, and for example, acetic acid, propionic acid, lactic acid, or the like is preferable.

**[Table 1]**

| (Basal medium) | |
|---|---|
| Component | Component concentration (w/v%) |
| (NH₄)₂SO₄ | 0.03 |
| KH₂PO₄ | 0.05 |
| MgSO₄·7H₂O | 0.02 |
| NaCl | 0.05 |
| NaHCO₃ | 0.02 |
| Yeast extract | 0.001 |

Examples of the modified MYS medium and the growth medium include media having the compositions shown in Tables 2 and 3 below.

**[Table 2]**

| (Composition of modified MYS medium) | |
|---|---|
| Component | Concentration |
| Sodium malate | 3.6 g/l |
| Yeast extract | 0.5 g/l |
| (NH₄)₂SO₄ | 1.0 g/l |
| KH₂PO₄ | 1.0 g/l |
| MgCl₂·6H₂O | 0.2 g/l |
| NaCl | 0.2 g/l |
| CaCl₂·2H₂O | 0.045 g/l |
| EDTA-2Na | 2 mg/l |
| FeSO₄·7H₂O | 2 mg/l |
| H₃BO₃ | 0.1 mg/l |
| CoCl₂·6H₂O | 0.1 mg/l |
| ZnCl₂ | 0.1 mg/l |
| MnCl₂·4H₂O | 0.1 mg/l |
| Na₂MoO₄·2H₂O | 0.02 mg/l |
| NiCl₂·6H₂O | 0.02 mg/l |
| CuCl₂·2H₂O | 0.01 mg/l |
| Na₂SeO₃ | 0.001 mg/l |
| Vitamin B₁-HCl | 0.5 mg/l |
| Nicotinic acid | 0.5 mg/l |
| p-aminobenzoate | 0.3 mg/l |
| Vitamin B₁₂ | 0.05 mg/l |
| Vitamin B₆-HCl | 0.1 mg/l |
| Vitamin H | 0.05 mg/l |

**[Table 3]**

| (Composition of growth medium) | |
|---|---|
| Component | Concentration |
| Sodium acetate | 3 g/l |
| Sodium lactate | 3 g/l |
| Sodium butyrate | 3 g/l |
| Sodium chloride | 5 g/l |
| L-glutamic acid | 0.17 g/l |
| K₂HPO₄ | 1 g/l |
| KH₂PO₄ | 1.5 g/l |
| EDTA | 20 mg/l |
| CaCl₂ | 70 mg/l |
| H₃BO₃ | 3 mg/l |
| COCl₂·6H₂O | 0.95 mg/l |
| ZnSO₄·7H₂O | 0.24 mg/l |
| Cu(NO₃)₂·3H₂O | 0.04 mg/l |
| NiCl₂·6H₂O | 0.02 mg/l |
| MgSO₄·7H₂O | 0.2 g/l |
| MnSO₄·5H₂O | 2 mg/l |
| Na₂MoO₄·2H₂O | 1 mg/l |
| FeSO₄·7H₂O | 20 mg/l |
| Biotin | 0.05 mg/l |
| VitaminB₁-HCl | 5 mg/l |
| Nicotinic acid | 5 mg/l |
| Yeast extract | 0.02 g/l |

In the above culture, the temperature range is not particularly limited, and is, for example, 23 to 39°C, or 30°C.

In the above-mentioned culture, the pH range is not particularly limited, and is, for example, pH 5.5 to 8.5, 6.0 to 8.5, or 7.0.

The culturing may be performed, for example, under aerobic conditions, or under anaerobic conditions, and is preferably performed under anaerobic conditions, although not particularly limited. The light condition at the time of culturing is not particularly limited, and may be, for example, a dark condition or an illumination condition, but is preferably under an illuminance of 2,000 lux to 10,000 lux. The culturing may be performed, for example, in a hermetically illuminated culture vessel. In addition, the culture medium may be cultured while being stirred by using a stirring device provided in the hermetically-illuminated culture vessel.

The culturing time is not particularly limited, and may be, for example, until the growth of the BP0899 strain reaches a stationary phase. The incubation time may be, for example, 72 hours if BP0899 strain is under incubation conditions that the growth of the BP0899 reachs a stationary phase within about 72 hours.

As described above, the base sequence of the 16S rRNA of the BP0899 strain is preferably the base sequence represented by SEQ ID NO: 1.

The base sequence of the 16S rRNA can be determined by extracting DNAs from the BP0899 strains isolated and cultured by, for example, the methods described above and the like, and using primers and the like. The method of extracting the DNA and determining the base sequence can be, for example, a conventional method, and is not particularly limited. The primer is not particularly limited, and for example, the following primer and the like can be cited.

### Primer

9F (SEQ ID NO:2) 5'-GAGTTTGATCCTGGCTCAG-3'
339F (SEQ ID NO: 3) 5'-CTCCTACGGGAGGCAGCAG-3'
785F (SEQ ID NO:4) 5'-GGATTAGATACCCTGGTAGTC-3'
1099F (SEQ ID NO: 5) 5'-GCAACGAGCGCAACCC-3'
536R (SEQ ID NO: 6) 5'-GTATTACCGCGGCTGCTG-3'
802 R (SEQ ID NO: 7) 5'-TACCAGGGTATCTAATCC-3'
1242R (SEQ ID NO: 8) 5'-CCATTGTAGCACGTGT-3'
1541R (SEQ ID NO:9) 5'-AAGGAGGTGATCCAGCC-3'

The culture of the BP0899 strain includes, for example, a bacterial cell of the BP0899 strain, a culture supernatant of the BP0899 strain, a bacterial cell extract of the BP0899 strain, and the like, and is not particularly limited.

The culture may be, for example, a treatment product of the bacterial cell, a treatment product of the culture supernatant, a treatment product of the bacterial cell extract, or the like, and is not particularly limited. The treatment product is not particularly limited, and may include, for example, a concentrate, a dried product, a lyophilized product, a solvent treatment product, a surfactant treatment product, an enzyme treatment product, a protein fraction, an ultrasonic treatment product, a grinding treatment product, and the like of the culture. The culture may be, for example, a mixture of the bacterial cell, the culture supernatant, the bacterial cell extract, the bacterial cell treated product, the culture supernatant treated product, the bacterial cell extract treated product, and the like. The mixture can be mixed in any combination and ratio, and is not particularly limited. The combination is not particularly limited, and may include, for example, a mixture of the bacterial cell and the culture supernatant.

An example of the crude extraction step is shown in the flowchart of FIG. 1. As shown in FIG. 1, the crude extraction step of the present example includes a decolorization process (step S11), an extraction process (step S12), and a filtration process (step S13).

### (1) Decolorization process (step S11)

First, pigment decolorization process is performed on the the Rhodobacter azotoformans BP0899 (contract number NITE BP 644) or cultures thereof. The decolorization process of the pigment is not particularly limited, and for example, a decolorization process with an organic solvent. Examples of the organic solvent include acetone, methanol, chloroform, and mixed solvents thereof. The decolorization process can be performed, for example, by mixing the bacterial cell or the culture with the organic solvent. Specifically, for example, 25 mL to 150 mL of acetone is added to 10 g to 60 g of the lyophilized bacteria of the BP0899 strain in a beaker, and the mixture is stirred thoroughly using a stirrer. Next, the supernatant of the stirred solution is transferred to a 50 mL conical tube, centrifuged at 2,000 rpm to 5,000 rpm for 5 minutes to 10 minutes, the obtained supernatant is removed, 20 mL to 40 mL of acetone is added to the precipitate, and the precipitate is returned to the beaker. This procedure is repeated until the color (brown) of the pigment of the BP0899 strain is not visually observed, and then the precipitate is dried under reduced pressure using an aspirator to a constant weight to obtain a dried bacterial cell decolorized.

### (2) Extraction process (step S12)

Next, the bacterial cells or cultures after the decoloring treatment are treated with an organic solvent that insolubilizes the proteins, and the proteins are removed. Examples of the organic solvent include phenol and the like. In the extraction process, for example, the bacteria or culture is mixed with the organic solvent and the aqueous solvent, the protein insolubilized by the organic solvent is distributed to the organic solvent phase, and the target compound is distributed to the aqueous solvent phase. Specifically, for example, water for injection is added to 10 g to 60 g of the decolored dried bacteria in the beaker so that the concentration of the decolored dried bacteria is 60 mg/mL to 90 mg/mL. Next, 90% phenol is added in an equal amount to the water for injection, and the mixture is stirred on a hot stirrer at 65°C to 70°C for 20 minutes to 40 minutes, and this is used as the first extraction. Then, after cooling the stirred solution to 10°C or lower, the solution is separated into a phenol phase and an aqueous phase by centrifugation using a centrifuge tube under the conditions of 8,000 rpm to 20000 rpm, 20 minutes to 60 minutes, and 2°C to 10°C, and the obtained aqueous phase is collected in a 50 mL conical tube, and an amount of water for injection equivalent to the collected aqueous phase is added to the phenol phase remaining in the centrifuge tube, and the same operation as the first extraction is repeated (second extraction). In addition, the same procedure as the first extraction is repeated (the third extraction). Thus, 500 mL to 1,000 mL of the aqueous phase obtained by extraction three times is collected.

### (3) Filtration process (step S13)

Next, the aqueous phase obtained by the extraction process is subjected to a filtration process to remove the organic solvent such as phenol used in the extraction process. Examples of the filtration include ultrafiltration and the like. The fractional molecular weight in the filtration is, for example, 7,000, and it is preferable to remove molecules less than the fractional molecular weight. Specifically, for example, dialysis is performed by placing the recovered aqueous phase in a dialysis tube having a molecular weight fraction of 7,000, and setting the external solution to 1 L to 10 L of distilled water. The dialysis is repeated until no absorption of light at 270 nm, which is the absorption wavelength of phenol, is observed in the external solution, and the internal solution is recovered as a crude extract containing the novel compound of the present invention.

### [Purification step]

An example of the purification step is shown in the flowchart of FIG. 2. As illustrated, the purification step of the present example includes an enzyme treatment process (step S21), an extraction process (step S22), and a filtration process (step S23).

### (1) Enzyme treatment process (step S21)

The crude extract containing the novel compound of the present invention obtained in the crude extraction step is subjected to an enzyme treatment. The enzyme treatment is not particularly limited, and may include, for example, treatment with a nuclease or treatment with a protease, and may be either treatment or both treatments. In the latter case, the order is not particularly limited, and, for example, after treatment with a nuclease, treatment with a protease can be performed.

First, the crude extract is treated with a nuclease. The nuclease is not particularly limited, and may be, for example, an ribonuclease or a deoxyribonuclease. The ribonucleases are not particularly limited, and for example, Ribonuclease A manufactured by Sigma Corporation, Ribonuclease A manufactured by Wako Pure Chemical Industries Co., Ltd., Ribonuclease A manufactured by Roche Corporation, and the like may be used. The deoxyribonucleases are not particularly limited, and for example, Deoxyribonuclease I manufactured by Sigma Corporation, Deoxyribonuclease I manufactured by Wako Pure Chemical Industries Co., Ltd., Deoxyribonuclease I manufactured by Roche Corporation, and the like may be used. Specifically, for example, 0.2 mg/mL to 1 mg/mL of ribonuclease and 1 µg/mL to 10 µg/mL of deoxyribonuclease are added to the crude extract, and incubated at 30°C to 40°C for 4 hours to 24 hours.

The crude extract is then treated with proteases. The proteases are not particularly limited, and for example, Proteinase K manufactured by Sigma Corporation, Proteinase K manufactured by Wako Pure Chemical Industries, Ltd., Proteinase K manufactured by Roche Corporation, and the like may be used. Specifically, for example, 100 µg/mL to 300 µg/mL of protease is added to the crude extract, and incubated at 40°C to 50°C for 2 hours to 24 hours.

### (2) Extraction process (step S22)

The crude extract is then treated with an organic solvent that insolubilizes the protein to remove the protein. Examples of the organic solvent include phenol and the like. Specifically, for example, the extract after the enzyme treatment process is centrifuged under the conditions of 2,000 rpm to 5,000 rpm and 20 minutes to 60 minutes. Then, of the obtained precipitate fraction of about 1 mL to 10 mL and the supernatant fraction of about 50 mL to 100 mL, the precipitate fraction is placed in an ultrafiltration tube having a molecular weight of 50,000 to 100,000, the external solution is set to be 5 mL to 15 mL of distilled water, and ultrafiltration is performed. To the obtained internal solution, 10 mL to 60 mL of water for injection and 10 mL to 60 mL of 90% phenol are add, the resultant is stirred on a hot stirrer at 65°C to 70°C for 20 minutes to 40 minutes, and the obtained solution is used as the first extraction. Then, after cooling the stirred solution to 10°C or lower, the solution is separated into a phenol phase and an aqueous phase by centrifugation using a centrifuge tube under the conditions of 8,000 rpm to 20,000 rpm, 20 minutes to 60 minutes, and 2°C to 10°C, and the obtained aqueous phase is collected in a 50 mL conical tube, and an amount of water for injection equivalent to the collected aqueous phase is added to the phenol phase remaining in the centrifuge tube, and the same operation as the first extraction is repeated (second extraction). In addition, the same procedure as the first extraction is repeated (the third extraction). In this way, a total of 60 mL to 120 mL of the aqueous phase of the three extraction operations is recovered.

### (3) Filtration process (step S23)

Next, the aqueous phase obtained by the extraction process is subjected to a filtration process to remove the organic solvent such as phenol used in the extraction process. Examples of the filtration include ultrafiltration and the like. The fractional molecular weight in the filtration is, for example, 50,000 to 100,000, and it is preferable to remove molecules less than the fractional molecular weight. Specifically, for example, the recovered aqueous phase is put into a dialysis tube having a molecular weight fraction of 7,000, and dialysis is performed for 24 hours to 96 hours with 0.5 L to 1 L of distilled water as the external liquid. The obtained internal solution is placed in an ultrafiltration tube with a molecular weight fraction of 50,000 to 100,000, the external solution is set to be 5mL to 15mL of distilled water, and ultrafiltration is performed. The obtained internal liquid is freeze-dried to obtain a compound represented by Formula (A) or Formula (B) or a salt thereof, which is a novel compound of the present invention.

### Examples

Next, examples of the present invention will be described. However, the present invention is not limited to the following examples. Commercial reagents were used based on these protocols unless otherwise indicated.

### [Example 1]

A compound represented by Formula (A) and a compound represented by Formula (B) were prepared by the following method.

### [1. Crude extraction step]

### (1-1) Decolorization process (Step S11).

To 20.03 g of the lyophilized bacteria of the above BP0899 strain in beakers, 50 ml of acetone was added, and the mixture was stirred for 10 minutes using a stirrer. Next, the supernatant of the stirred solution was transferred to a 50 mL conical tube, centrifuged at 2,000 rpm for 5 minutes, the obtained supernatant was removed, 20 mL of acetone was added to the precipitate, and the precipitate was returned to the beaker. This procedure was repeated until no brown color of the pigment of the BP0899 strain was visually observed. The decolorized precipitate was dried under reduced pressure using an aspirator to a constant weight to obtain a decolorized dried bacterial cell.

### (1-2) Extraction process (step S12)

Water for injection was added to 16 g of the decolored dried bacteria in a beaker so that the concentration of the decolored dried bacteria was 75 mg/mL. Next, 90% phenol was added in an equal amount to the water for injection, and the mixture was stirred on a hot stirrer at 65°C to 70°C for 30 minutes, and this was used as an initial extraction. Then, the stirred solution was cooled to 10°C or lower, and then centrifuged at 15,000 rpm for 40 minutes at 4°C using a centrifuge tube to separate the phenol phase and the aqueous phase, and the obtained aqueous phase was collected in a 50 mL conical tube, and an amount of water for injection equivalent to the collected aqueous phase was added to the phenol phase remaining in the centrifuge tube, and the same operation as the first extraction was repeated (second extraction). In addition, the same procedure as the first extraction was repeated (third extraction). In this way, 450 mL of the aqueous phase of three extraction runs were collected.

### (1-3) Filtration process (Step S13)

The collected 450 mL of the aqueous phase was put into a dialysis tube having a molecular weight fractionation of 7,000, and dialysis was performed with the external liquid being 2.5 L of distilled water. The dialysis was carried out 22 times until no absorption of light at 270 nm, which is an absorption wavelength of phenol, was observed in the external solution, and 75 mL of the internal solution, which was a crude extract containing the compound represented by Formula (A) and the compound represented by Formula (B), was collected.

### [2. Purification step]

### (2-1) Enzyme treatment process (step S21)

First, 0.5 mg/mL of RNA-degrading enzyme (trade name: ribonuclease A manufactured by Sigma Corporation) and 5 µg/mL of DNA-degrading enzyme (Deoxyribonuclease I manufactured by Sigma Corporation) were added to the crude extraction solution containing the compound represented by Formula (A) and the compound represented by Formula (B) obtained in the crude extraction step, and the mixture was incubated at 37°C for 6 hours. Next, 200 µg/mL of proteases (Proteinase K manufactured by Sigma Corporation) were added to the crude extract, incubated at 50°C for 4 hours, and centrifuged at 3,000 rpm for 30 minutes.

### (2-2) Extraction process (Step S22).

Of the precipitated fraction of about 3 mL or less and the supernatant fraction of about 72 mL obtained by centrifugation in the enzyme treatment process, the precipitated fraction was placed in an ultrafiltration tube having a molecular weight of 100,000, and the external liquid was set to be 15 mL of distilled water, and ultrafiltration was performed. To the obtained internal solution, 30 mL of water for injection and 30 mL of 90% phenol were added, and the mixture was stirred on a hot stirrer at 65°C to 70°C for 30 minutes, and this was used as an initial extraction. Then, the stirred solution was cooled to 10°C or lower, and then centrifuged at 15,000 rpm for 40 minutes at 4°C using a centrifuge tube to separate the phenol phase and the aqueous phase, and the obtained aqueous phase was collected in a 50 mL conical tube, and an amount of water for injection equivalent to the collected aqueous phase was added to the phenol phase remaining in the centrifuge tube, and the same operation as the first extraction was repeated (second extraction). In addition, the same procedure as the first extraction was repeated (third extraction). Thus, 80 mL of the aqueous phase of three extraction runs was collected.

### (2-3) Filtration process (Step S23)

The recovered aqueous phase was put into a dialysis tube having a molecular weight fractionation of 7,000, and dialysis was performed for 72 hours with 1 L of distilled water as the external liquid. The obtained internal solution was put into an ultrafiltration tube having a molecular weight of a fraction of 100,000, and the external solution was set to be 15 mL of distilled water, and ultrafiltration was performed. The obtained internal solution was freeze-dried to obtain 164.53 mg of a purified product.

### [Example 2]

The purification material was subjected to mass spectrometry and nuclear magnetic resonance (NMR) to identify its structures.

### (1) Mass spectrometry of the decomposition product of the purified product

The above-mentioned purification product was decomposed in the following manner to prepare a decomposition product of the above-mentioned purified product. First, the purified product was dissolved in 0.1 mol/L hydrochloric acid to a concentration of 10 mg/mL, and the dissolved solution was heated in a water bath for 90 minutes to obtain a precipitate and a colorless clear supernatant. Next, the precipitate was collected, subjected to chloroform extraction, and a chloroform fraction was collected. Chloroform was added to the chloroform fraction to a concentration of 38 mg/mL, and 6 µL of this solution was subjected to thin layer chromatography (TLC). As the TLC plate, a 10 cm x 10 cm silica gel 60F254 TLC plate (manufactured by Merck Corporation) was used, and as the developing solvent, a solution of chloroform:methanol:distilled water:triethylamine = 30:13:2:0.1 (volume ratio) was used. After development, the TLC plate was sprayed with 50% sulfuric acid to scrape off the gel corresponding to the most intensely stained spot, and dissolved again in the developing solvent. After the solvent was removed, fractions other than the solvent were collected and freeze-dried to obtain a decomposition product of the purified product (hereinafter referred to as "decomposition product").

Each of the above decomposition products was subjected to the following treatment to prepare four sample solutions: a methyl esterified product, a pyrrolididized product, a hydrolyzed methyl esterified product, and a hydrolyzed pyrrolididized product.

### <Preparation of methyl esterified product>

(I) To 0.1 mL of the chloroform solution prepared so that the decomposition product concentration was 30 mg/mL, 1 mL of a 0.2 mg/mL BHT (dibutylhydroxytoluene) chloroform solution was added and dried.
(II) To the dried product, 1 mL of 5% hydrochloric acid and methanol was added and reacted at 85°C for 24 hours.
(III) After the reaction was allowed to cool, 1 mL of hexane and 0.5 mL of water were added thereto, and the hexane phase was collected.
(IV) The collected hexane phase was removed by evaporation under a stream of nitrogen and redissolved in 1 mL of chloroform to obtain a sample solution of a methyl ester product.

### <Preparation of pyrrolididized product>

(I) 200 µL of the sample solution of the methyl esterified product was collected in a glass test tube.
(II) To the glass test tube, 400 µL of pyrrolidine and 40 µL of acetic acid were added and reacted at 100°C for 30 minutes.
(III) After completion of the reaction, 2 mL of dichloromethane and 2 mL of a 5% aqueous solution of acetic acid were added to the reaction mixture, the mixture was shaken, and then the dichloromethane phase was recovered.
(IV) After the solvent was removed from the recovered dichloromethane phase under a stream of nitrogen, it was redissolved in 200 µL of chloroform to obtain a sample solution of a pyrrolididized product.

### <Preparation of hydrolyzed methyl esterified product>

(I) The decomposition product was subjected to the same treatment as in (I) in the preparation of the methyl esterified product to obtain a dried product.
(II) To the dried product, 1 ml of 0.5 mol/L NaOH methanol was added, and the mixture was reacted at 50°C for 1 hour.
(III) After the reaction was allowed to cool, 1 mL of hexane and 0.5 mL of 1 mol/L hydrochloric acid test solution were added thereto, and the hexane phase was collected.
(IV) The recovered hexane phase was subjected to the same treatment as in (IV) in the preparation of the methyl esterified product to obtain a sample solution of the hydrolyzed methyl esterified product.

### <Preparation of hydrolyzed pyrrolididized product>

The hydrolyzed methyl esterified product was subjected to the same treatment as (I) to (IV) in the preparation of the pyrrolididized product to obtain a sample solution of the hydrolyzed pyrrolididized product.

### (1-1) GC-MS of methyl esterified product and hydrolyzed methyl esterified product

The methyl esterified product and the hydrolyzed methyl esterified product were subjected to GC-MS (Gas Chromatograph-Mass Spectrometry) analysis under the following conditions.

### <GC-MS Conditions>

Equipment: JMS-700V (manufactured by Nippon Electronics Co., Ltd.)
Column: SPB-1 30m x 0.25mm Film thickness 0.25 µm
Column temperature: 50°C (held for 1 minute) → 300°C (+8°C/minute, held for 30 minutes)
Injection port temperature :250°C
Detector: Flame Ionization Detector (FID) 300°C
Injection volume: 1 µL (splitless injection)
Carrier gas: helium (linear velocity: 30 cm/sec, constant flow mode)
Detector :MS
   Ionization method: EI (Electron Ionization)
   Ionizing current :300 µA
   Ionization energy: 70 eV
   Ionization room temperature: 300°C
   Electron accelerating voltage :10 kV
   Scanning zone :m/z = 35 to500 (sec/scan)

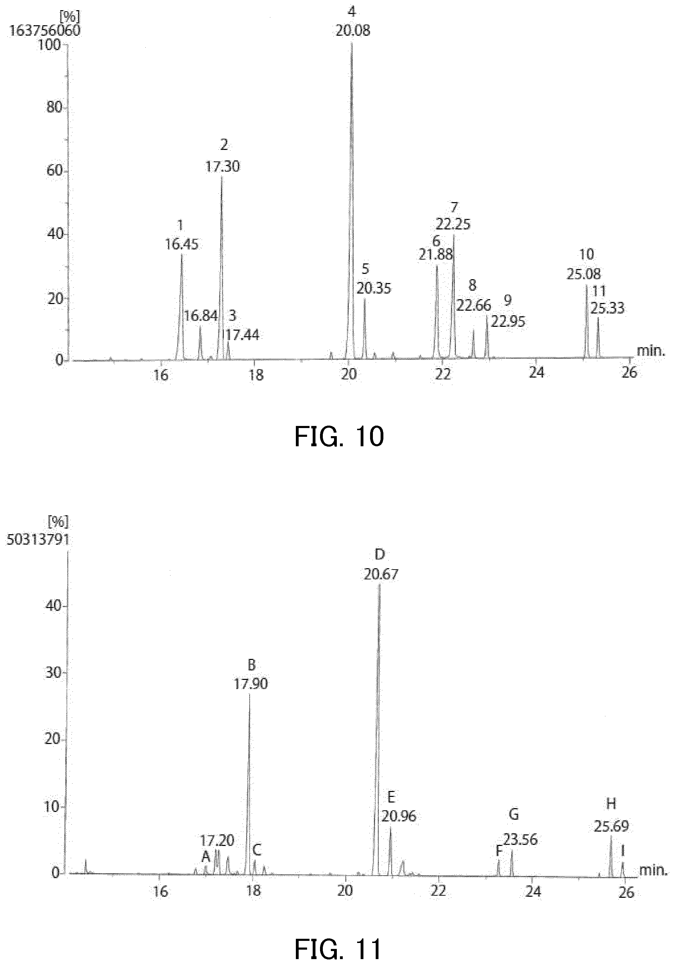

The results are shown in FIGs. 10 and 11. FIG. 10 is a mass spectrum obtained by subjecting the methyl esterified product to GC-MS analysis, and FIG. 11 is a mass spectrum obtained by subjecting the hydrolyzed methyl esterified product to GC-MS analysis. In FIGs. 10 and 11, the vertical axis represents the detection intensity, and the horizontal axis represents the detection time (min).

As shown in FIG. 10, peaks 1 to 11 were obtained from the methyl esterified product. Among these, the compounds of peaks 1, 2, 4, 6 and 7 having high detection intensities were further subjected to GC-MS analysis under the same conditions as described above. Structures with mass spectra similar in form to the mass spectra obtained were library searched by Auto Mode of databases (The NIST MassSpectral Seach Program for the NIST/EPA/NIH MassSpectral Library). As a result, the compounds of peaks 1, 2, 4, and 7 showed a high degree of similarity to the compounds of Formulae (2) to (5), respectively. The inventors have also discovered that the mass spectrum of the peak 6 compound is closely similar to the mass spectrum of the 3-oxo-tetradecanoic acid methyl ester described in "Strittmatter W.et al (1983), Journal of Bacteriology, Vol. 155, No. 1, p. 153-p. 158, Fig2" Strittmatter et al. Thus, the compound at peak 6 was estimated to be 3-oxo - tetradecanoic acid methyl ester of Formula (6).

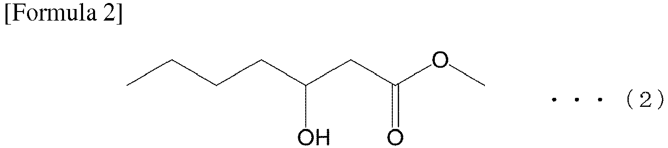

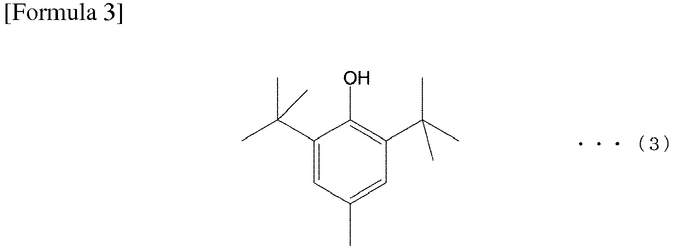

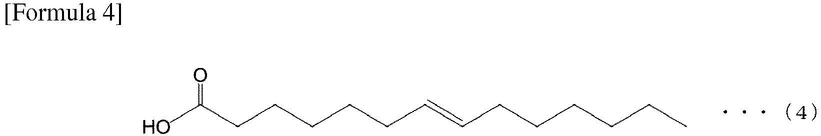

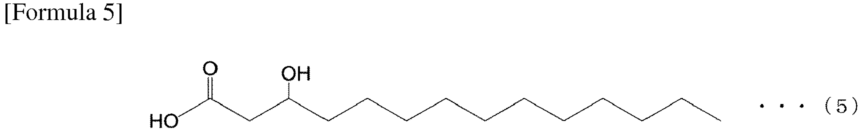

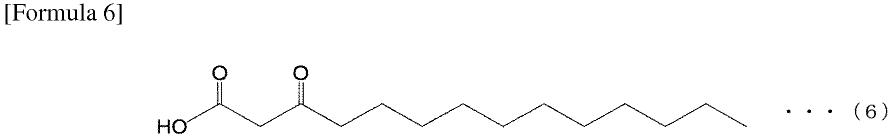

As shown in FIG. 11, peaks A to I were obtained from the hydrolyzed methyl esterified product. Here, when comparing the mass spectrum of FIG. 10 and the mass spectrum of FIG. 11, peaks A, B, and D were confirmed as peaks corresponding to peaks 1, 2, and 4 of FIG. 10, respectively, in FIG. 11. Each of these peaks A, B and D was further subjected to GC-MS analysis under the same conditions as described above. Structures with mass spectra similar in shape to the mass spectra obtained were searched using the database. As a result, peaks A, B, and D showed high similarity to the compounds of Formula (7), Formula (3), and Formula (4), respectively.

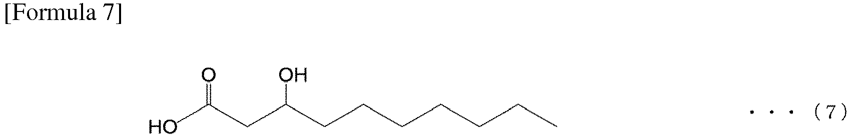

Note that the peaks corresponding to peaks 6 and 7 in FIG. 10 were not identified in FIG. 11. This is probably due to the following reasons. That is, FIG. 10 shows the result of subjecting the decomposition product to GC-MS analysis without hydrolysis, whereas FIG. 11 shows the result of subjecting only a separated product which is obtained by hydrolyzing the decomposition product to GC-MS analysis. Therefore, the peak identified in FIG. 11 is considered to be the peak of the compound having the ester bond hydrolyzed in the decomposition product. Thus, the compounds of peaks 6 and 7 of FIG. 10 for which the corresponding peaks were not identified in FIG. 11 are estimated to be compounds that do not have the ester bond hydrolyzed in the decomposition product.

The results of FIG. 10 and FIG. 11 are summarized and the compounds of peaks 1, 2, 4 and 7 having high detection intensities in FIG. 10 are estimated as shown in the following description and Table 4. First, as described above, the compound of the peak 1 in FIG. 10 was estimated to be the compound of the Formula (2) as a result of the library search. However, when the compound of peak 1 is the compound of Formula (2), a peak should be observed at a shorter retention time (i.e., to the left) than peak 1 in FIG. 10, resulting in inconsistency. On the other hand, the compound of the peak A in FIG. 11 corresponding to the peak 1 in FIG. 10 was estimated to be the compound of the Formula (7) as a result of the library search as described above. When the compound of the peak A is a compound of the Formula (7), the result of the peak A in FIG. 11 is also consistent. Therefore, it was estimated that the compound of the peak 1 in FIG. 10 was the compound of the Formula (7). Next, as described above, the compound of the peak 2 in FIG. 10 was estimated to be BHT (dibutylhydroxytoluene) of the Formula (3) as a result of the library search. The compound of the peak B of FIG. 11 corresponding to the peak 2 of FIG. 10 was also estimated to be BHT (dibutylhydroxytoluene) of the Formula (3) as a result of the library search as described above, and the results agreed between FIG. 10 and FIG. 11. Therefore, the compound of the peak 2 in FIG. 10 was estimated to be BHT (dibutylhydroxytoluene) of the Formula (3), but this was estimated not to be a compound included in the decomposition product but to be BHT (dibutylhydroxytoluene) added in the preparation of the sample solution. The compound of the peak 4 in FIG. 10 was estimated to be the compound of the Formula (4) as a result of the library search as described above. The compound of the peak D in FIG. 11 corresponding to the peak 4 in FIG. 10 was also estimated to be the compound of the Formula (4) as a result of the library search as described above. Therefore, it was estimated that the compound of the peak 4 in FIG. 10 was the compound of the Formula (4). The discussion of the determination of the carbon-to-carbon double bond of a compound of Formula (4) is described in detail in (1-2) below. Furthermore, the compound of peak 7 in FIG. 10 was estimated to be the compound of Formula (5) as a result of the library search, as described above.

**[Table 4]**

| FIG. 10 (Methyl esterified product) | | FIG. 11 (Hydrolyzed methyl esterified product) | | Final estimated compound |
|---|---|---|---|---|
| Peak No. | Sequence No. | Peak No. | Sequence No. | |
| Peak 1 | (2) | Peak A | (7) | Formula (7) |
| Peak 2 | (3) | Peak B | (3) | Formula (3) (additive reagent BHT) |
| Peak 4 | (4) | Peak D | (4) | Formula (4) |
| Peak 6 | (6) | Not detected | | Formula (6) |
| Peak 7 | (5) | Not detected | | Formula (5) |

### (1-2) GC-MS of pyrrolididized product and hydrolyzed pyrrolididized product

Next, for the purpose of determining the position of the carbon-carbon double bond in the compound of Formula (4), the pyrrolididized product and the hydrolyzed pyrrolididized product were subjected to GC-MS analysis under the following conditions.

### <GC-MS Conditions>

Equipment: JMS-700V (manufactured by Nippon Electronics Co., Ltd.)
Column: SPB-1 30m x 0.25mm Film thickness 0.25 µm
Column temperature: 100°C (held for 1 minute) → 300°C (+10°C/minute, held for 30 minutes)
Injection port temperature :280°C
Detector: Flame Ionization Detector (FID) 300°C
Injection volume: 1 µL (splitless injection)
Carrier gas: helium (linear velocity: 30 cm/sec, constant flow mode)
Detector :MS
   Ionization method :EI
   Ionizing current :300 µA
   Ionization energy: 70 eV
   Ionization room temperature: 300°C
   Electron accelerating voltage :10 kV
   Scanning zone :m/z = 35 to 500 (sec/scan)

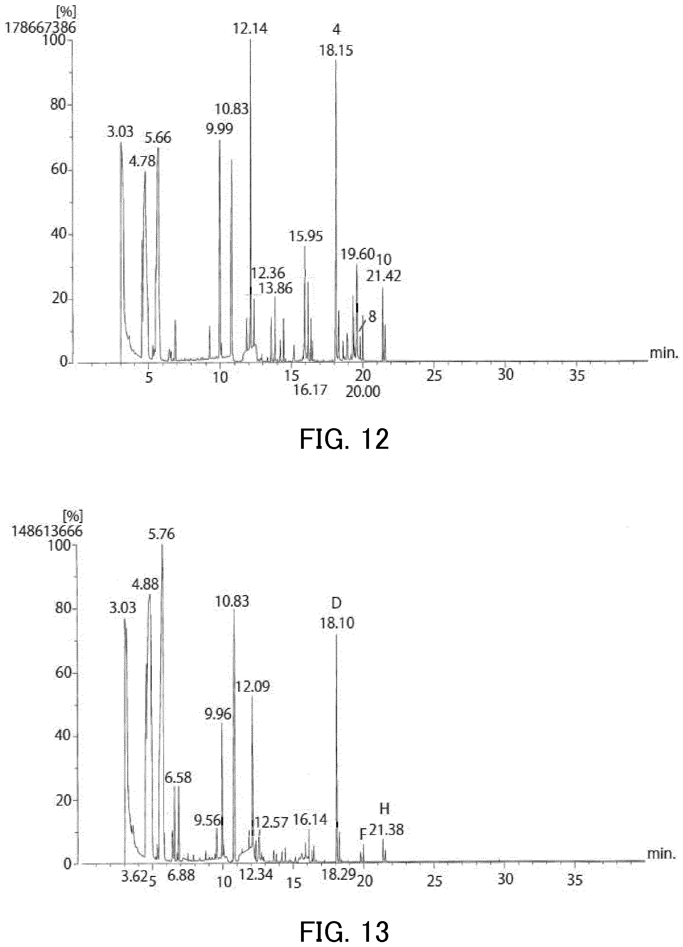

The results are shown in FIGs. 12 and 13. FIG. 12 is a mass spectrum obtained by subjecting the pyrrolididized product to GC-MS analysis, and FIG. 13 is a mass spectrum obtained by subjecting the hydrolyzed pyrrolididized product to GC-MS analysis. In FIGs. 12 and 13, the vertical axis represents the detection intensity, and the horizontal axis represents time (min).

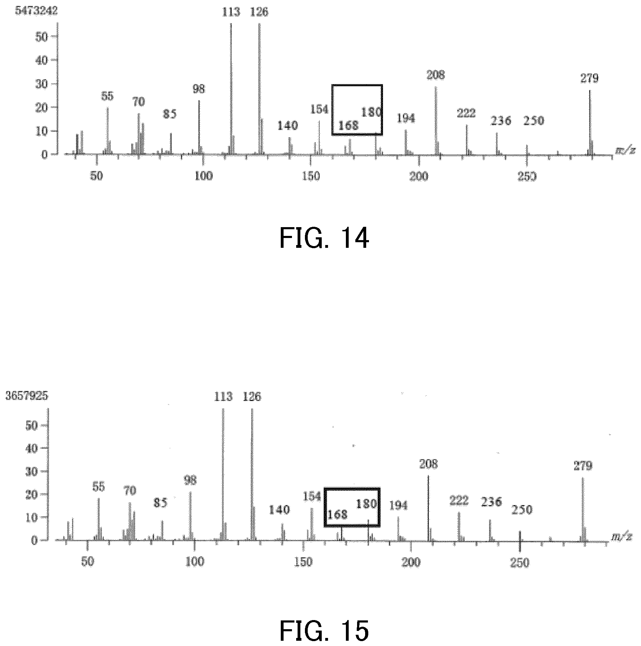

The compounds of peak 4 in FIG. 12 and peak D in FIG. 13 were further subjected to GC-MS analysis under the same conditions as described above. As a result, the mass spectrum shown in FIG. 14 was obtained from the compound of peak 4, and the mass spectrum shown in FIG. 15 was obtained from the compound of peak D. In FIGs. 14 and 15, the vertical axis represents the detection intensity, and the horizontal axis represents the m/z value. Here, as shown in Formula (8), the molecular weight is 140 when the compound is cleaved between the 4th and 5th carbons counted from a carbon to which hydrazine is bonded. Thus, if the compound is cleaved between the 7th and 8th carbons counted from a carbon to which hydrazine is bonded, m/z = 182 would be detected in the absence of a double bond. However, m/z = 180 was detected because of the double bond at this position. From the above, it was estimated that the carbon-carbon double bond in the compound of Formula (4) exists between the seventh and eighth carbons counting from the carbonyl group carbon.

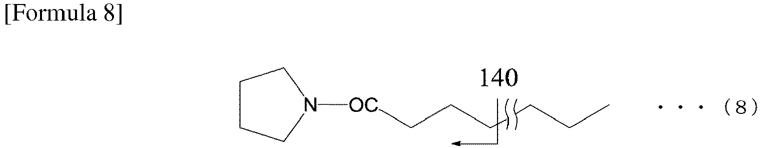

In summary of the results of (1-1) and (1-2) above, it was estimated that the decomposition product contained four compounds of Formulae (4) to (7). In addition, as described above, the compounds of Formula (5) and Formula (6) were estimated to be compounds having no ester bond because the peaks were not confirmed in FIG. 11 when only the substance obtained by hydrolyzing and separating the decomposition product was analyzed by GC-MS.

(2) Structural analysis of the decomposition product of the purified product Solvents were removed from 0.1 mL of the chloroform solution prepared so that the concentration of the decomposition product obtained in the above (1) was 30 mg/mL, and 600 µL of heavy DMSO was added to the chloroform solution, which was transferred to a 5 mm test tube, and used as a sample solution.

### <¹HNMR measurement and ¹³CNMR measurement>

The sample solution was subjected to ¹HNMR and ¹³CNMR measurements under the following measuring conditions.

### Measurement conditions:

Equipment: UNITY INOVA 500 type (manufactured by Varian)
Observation frequency: 499.8 MHz (¹H nucleus)
125.7 MHz (¹³C nucleus)
Solvents: heavy DMSO
Standard (*): Solvents: ¹H nucleus (2.49 ppm), ¹³C nucleus (39.7 ppm)
Temperature: set to 70°C
Measurements: ¹³CNMR; DEPT; NOESY; ROESY; COSY; TOCSY; HSQC; HMBC
* The values for chemical shifts in heavy DMSO at 70°C were as described in Alba et al. (Alba S.et al (2004), Glycobiology, vol. 14, no. 9, p. 805-p. 815).

### <³¹PNMR measurement>

A 3 mm tube containing 200 µL of 85% phosphoric acid was inserted into the 5 mm tube containing the sample solution. At this time, the observed signal derived from 85% phosphoric acid was adjusted to 0.000 ppm, and then the 3mm test tube was removed, and the sample solution was subjected to ³¹PNMR measurements under the following measurement conditions.

### Measurement conditions:

Equipment: UNITY INOVA 500 type (manufactured by Varian)
Observation frequency: 499.8 megahertz (³¹P nucleus)
Solvents: heavy DMSO
Baseline: 85% phosphoric acid (external standard): 0.000 ppm
Temperature: set to 25°C

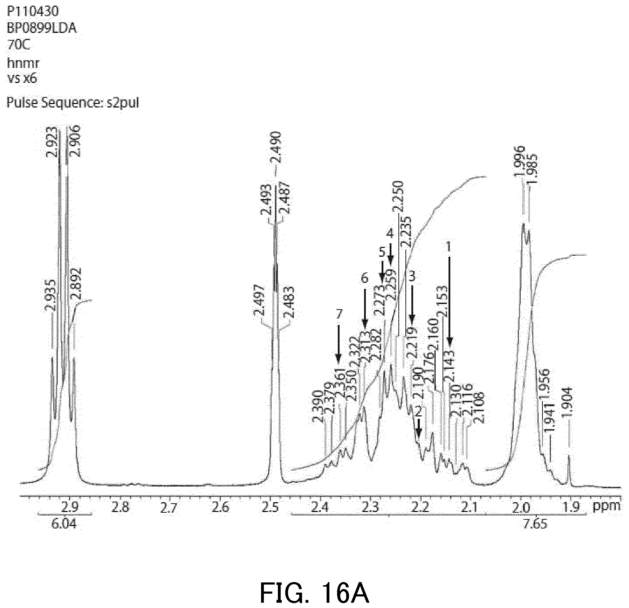

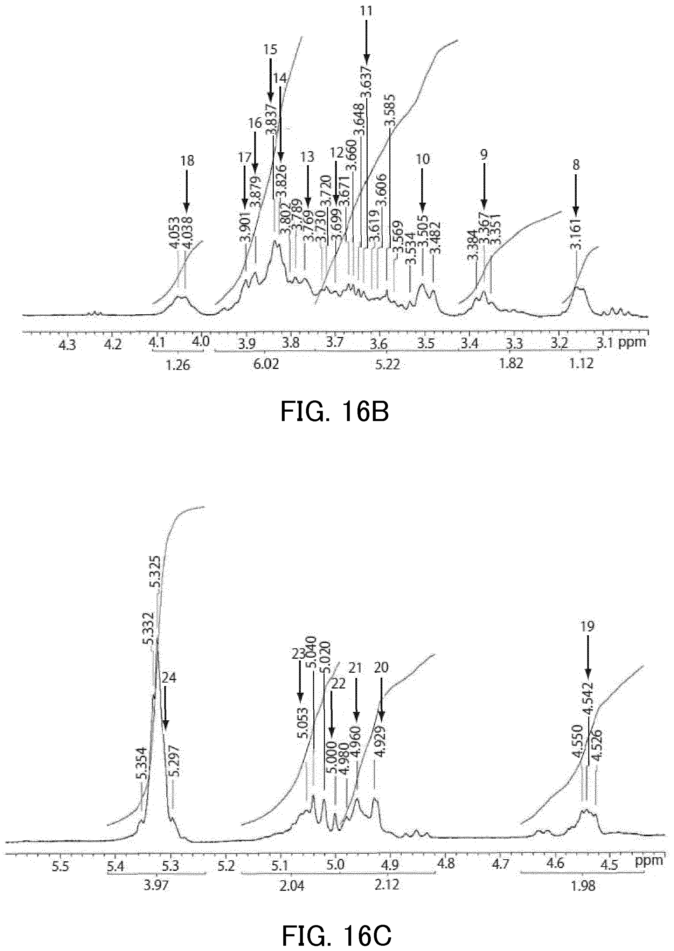

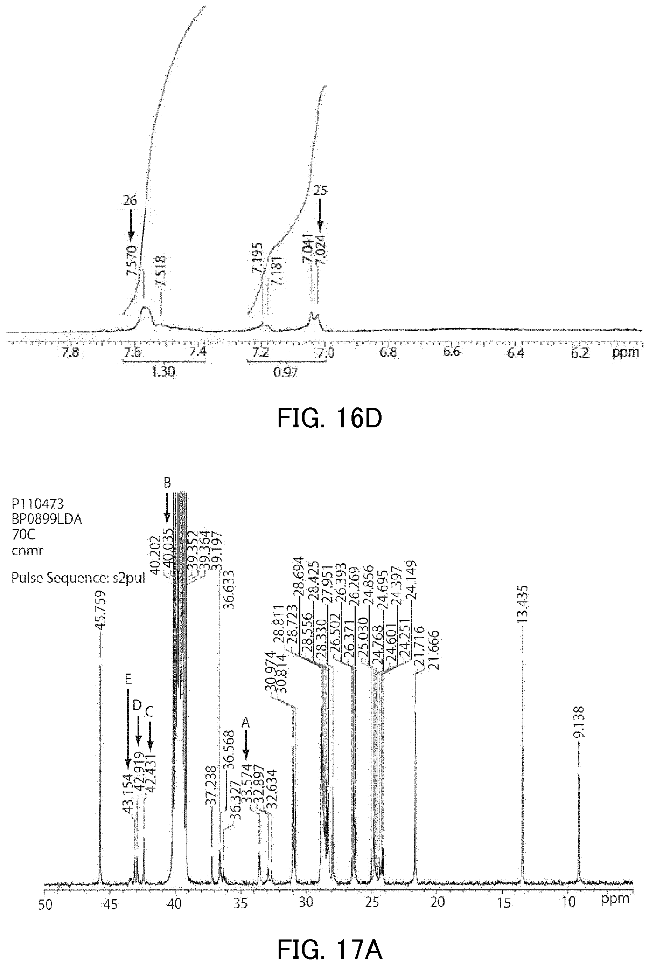

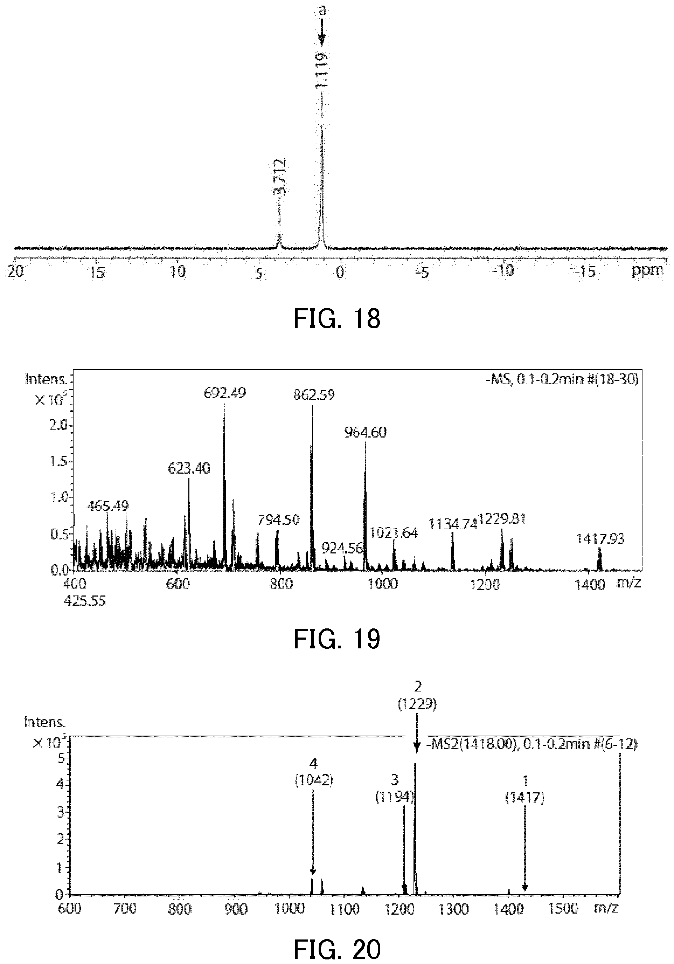

The effects are shown in FIGs. 16 to 18. FIGs. 16A-16D are spectra of ¹HNMR measurement, FIGs. 17A-17D are spectra of ¹³CNMR measurement, and FIG. 18 is spectra of ³¹PNMR measurement. In FIGs. 16 to 18, the vertical axis represents the detection intensity, and the horizontal axis represents the chemical shift value in ppm.

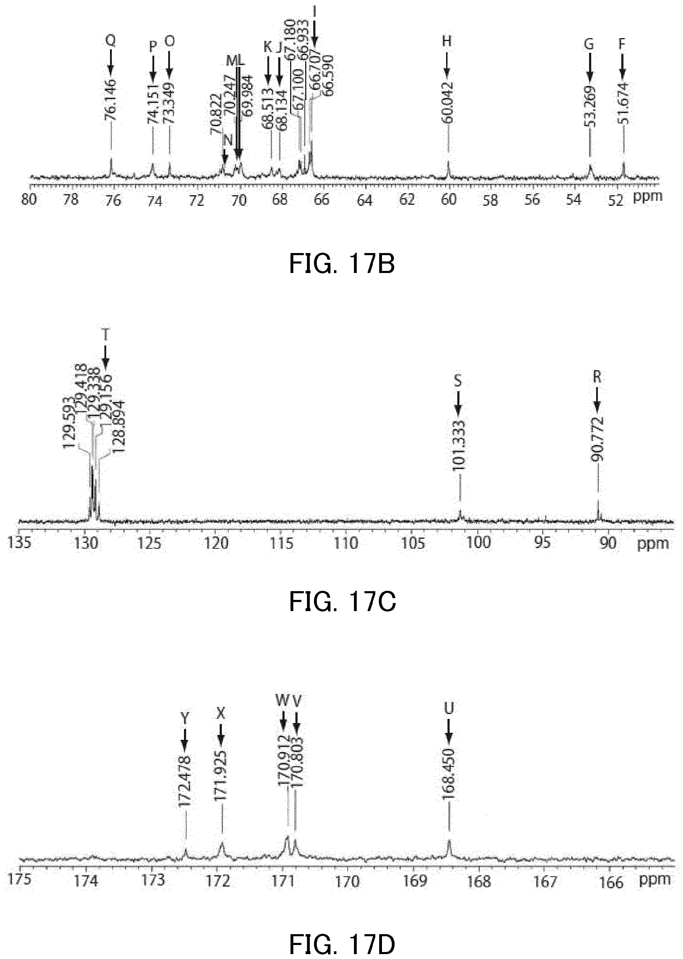

From the spectra of FIGs. 16 to 18, it was estimated that the decomposition product was a compound of the Formula (9) containing the four compounds and two molecules of glucosamine. In Formula (9), the four compounds bonded to two molecules of glucosamine are, in order from the left, a compound of Formula (7), a compound of Formula (5), a compound of Formula (4), a compound of Formula (7), and a compound of Formula (6). The numbers in Formula (9) correspond to the numbers of the peaks in the spectra of the ¹HNMR measurements of FIG. 16, the alphabets (uppercase letters) in Formula (9) correspond to the alphabets (uppercase letters) of the peaks in the spectra of the ¹³CNMR measurements of FIG. 17, and the alphabets (lowercase letters) in Formula (9) correspond to the alphabets (lowercase letters) of the peaks in the spectra of the ³¹PNMR measurements of FIG. 18.

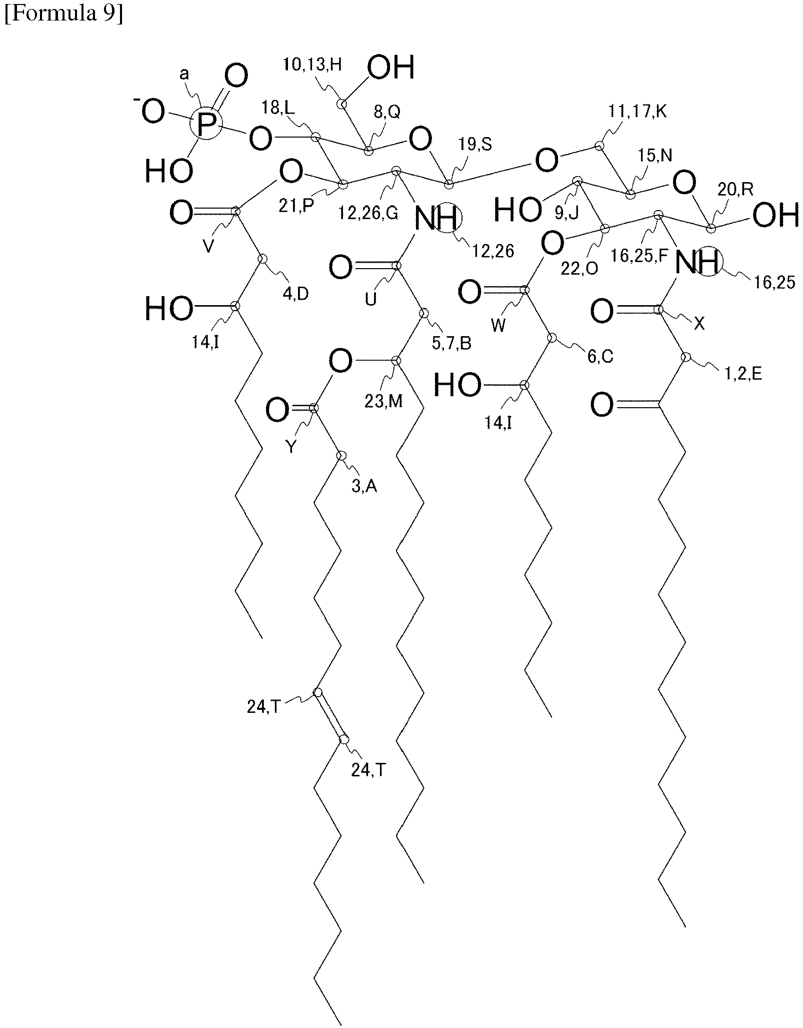

### (3) Mass spectrometry of the decomposition product of the purified product

To further confirm that the decomposition product is a compound of Formula (9), the following analysis was performed.

A chloroform solution prepared so that the concentration of the decomposition product obtained in the above (1) was 30 mg/mL was diluted by 40,000 times with methanol, thereby obtaining a sample solution. Then, the sample solution was subjected to GC-MS analysis under the following conditions.

### <GC-MS Conditions>

Equipment: amaZon ETD (Bruker Daltonics Co., Ltd.) with ESI interface
Measuring modes: ESI-IT-MS, Negative mode

The results are shown in FIG. 19. FIG. 19 is a mass spectrum obtained by subjecting the sample solution to GC-MS analysis. In FIG. 19, the vertical axis represents the detection intensity, and the horizontal axis represents the m/z value. As shown in FIG. 19, a plurality of peaks were confirmed, and the peak closest to the molecular weight of the compound of Formula (9), which is the estimated compound of the decomposition product, is the peak of m/z = 1417.93. Therefore, m/z = 1417.93 was determined to be the peak of the decomposition product, and this peak was further subjected to GC-MS/MS analysis under the same conditions.

The results are shown in FIG. 20. FIG. 20 is a mass spectrum obtained by subjecting the compound having a peak of m/z = 1417.93 in FIG. 19 to GC-MS/MS analysis. In FIG. 20, the vertical axis represents the detection intensity, the horizontal axis represents the m/z value, and the numerical value in parentheses represents the m/z value.

The following was deduced from FIG. 20. The compound of peak 1 was estimated to be the compound of Formula (9) as described above. The compound of peak 2 was estimated to be the compound in which a compound of Formula (7) having a molecular weight of about 188 was separated from the decomposition product because the difference value obtained by subtracting the m/z value (1229) of the peak 2 from the m/z value (1417) of the peak 1 was 188. The compound of peak 3 was estimated to be the compound in which a compound of Formula (4) having a molecular weight of about 223 was separated from the decomposition product because the difference value obtained by subtracting the m/z value (1194) of the peak 3 from the m/z value (1417) of the peak 1 was 223. The compound of peak 4 was estimated to be the compound in which two compounds of Formula (7) each having a molecular weight of about 188 were separated from the decomposition product because the difference value obtained by subtracting the m/z value (1042) of the peak 4 from the m/z value (1417) of the peak 1 was 375. Thus, the compounds obtained from separating the compound of Formula (7) or the compound of Formula (4) from the compound of Formula (9) was confirmed as peaks 2 to 4. From this, it can be confirmed that at least two of the compound of the Formula (7) and the compound of the Formula (4) are included in the decomposition product, and a result further supporting the estimated structural Formula (9) of the decomposition product estimated in the above (2) was obtained.

### (4) Mass spectrometry of the purified product

The purified product was hydrazinolysed by the process described in Leone et al. (Serena Leone et al,"Structural elucidation of the core-lipid A backbone from the lipopolysaccharide of Acinetobacter radioresistens S13,an organic solvent tolerant Gram-negative bacterium", Carbohydrate Research, April 10, 2006, Vol. 341, issue. 5, p. 582-590) to give 105.4 mg of hydrazine decomposition product. A solution obtained by dissolving the above-mentioned hydrazine decomposition product in distilled water to a concentration of 2.1 mg/mL and diluting it 200-fold with methanol was used as a sample solution and subjected to GC-MS analysis under the following conditions.

### <GC-MS Conditions>

Equipment: amaZon ETD (Bruker Daltonics Co., Ltd.) with ESI interface
Measuring modes: ESI-IT-MS, Negative mode

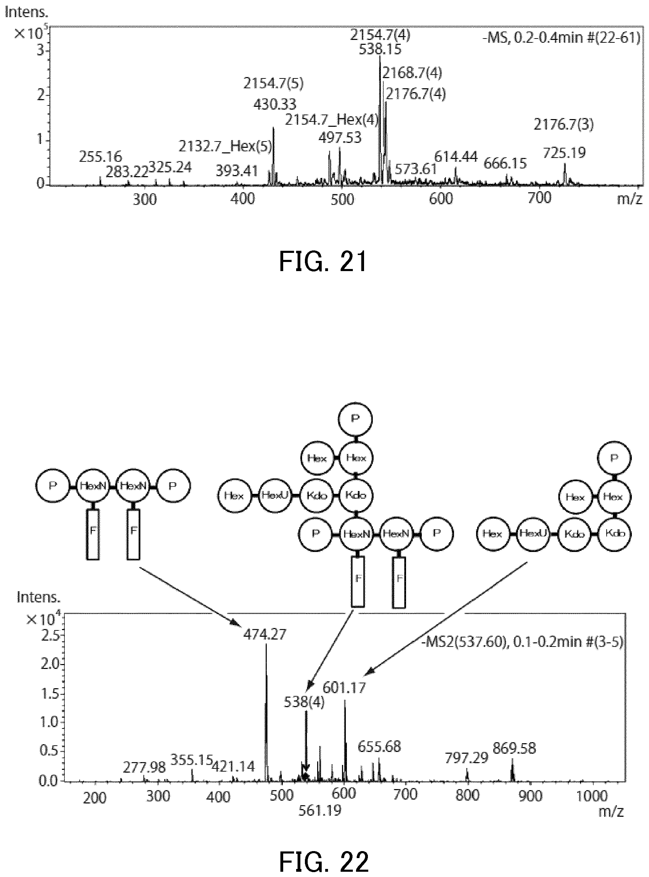

The results are shown in FIG. 21. FIG. 21 is a mass spectrum obtained by subjecting the sample solution to GC-MS analysis. In FIG. 21, the vertical axis represents the detection intensity, the horizontal axis represents the m/z value, and the numerical value in parentheses represents the valence of ions to be detected.

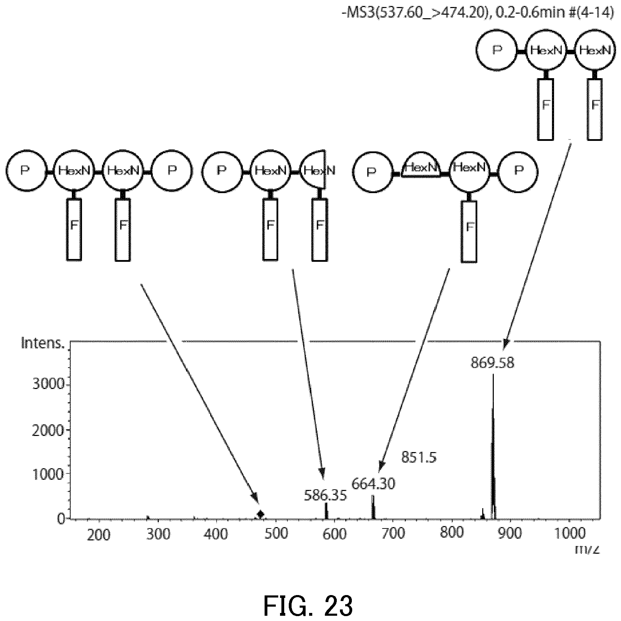

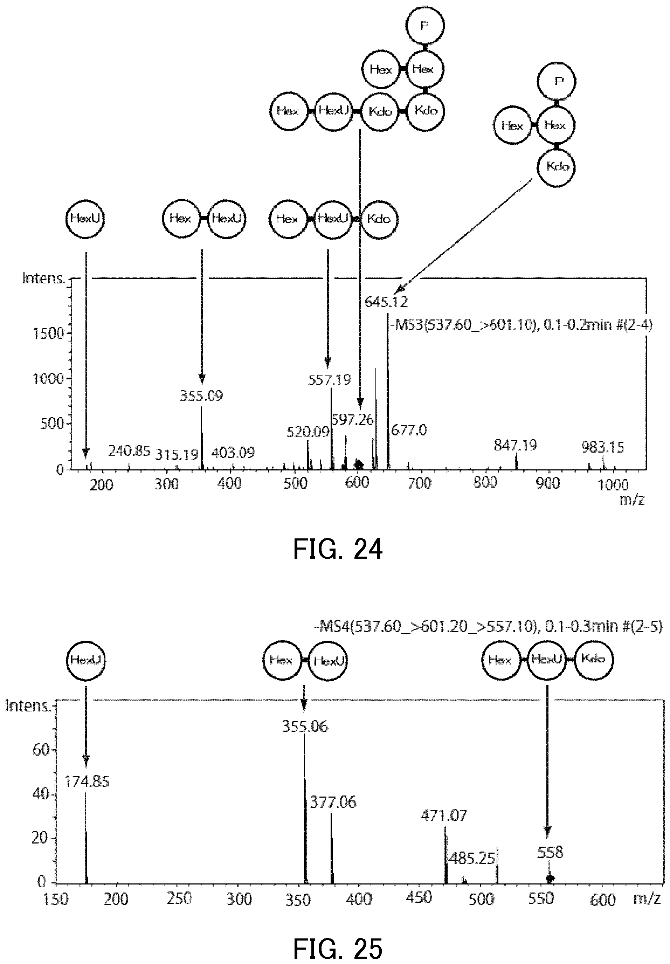

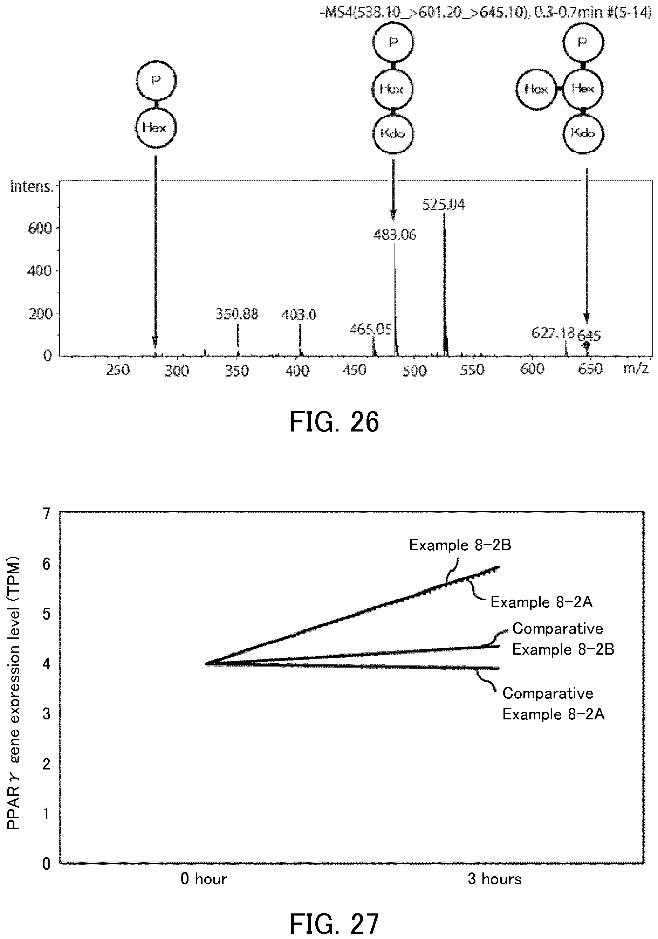

The compound having the largest peak (m/z = 538.15) among the plurality of peaks shown in FIG. 21 was subjected to GC-MS/MS analysis under the same conditions, and the mass spectrum shown in FIG. 22 was obtained. Further, of the plurality of peaks shown in FIG. 22, the compound having the peak of m/z = 474.27 and the compound having the peak of m/z = 601.17 were further subjected to GC-MS/MS/MS analysis under the same conditions, respectively, and mass spectra shown in FIGs. 23 and 24 were obtained. Further, of the plurality of peaks shown in FIG. 24, the compound having the peak of m/z = 557.19 and the compound having the peak of m/z = 645.12 were further subjected to GC-MS/MS/MS analysis under the same conditions, respectively, and the mass spectra shown in FIGs. 25 and 26 were obtained. In FIGs. 22 to 26, the vertical axis indicates the detection intensity, the horizontal axis indicates the m/z value, and the numerical values in parentheses indicate the valence of ions to be detected.

FIGs. 22 to 26 show schematic diagrams of compounds estimated to correspond to the respective peaks in addition to the mass spectra. In the above schematic diagram, P represents the structural formula of Formula (10), Hex represents the structural formula of Formula (11), Kdo represents the structural formula of Formula (12), HexU represents the structural formula of Formula (13), HexN represents the structural formula of Formula (14), and F represents any of the four compounds estimated in the above (2). In FIGs. 22 to 26, Hex is glucose (Glc).

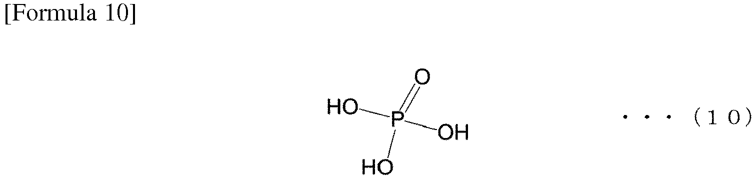

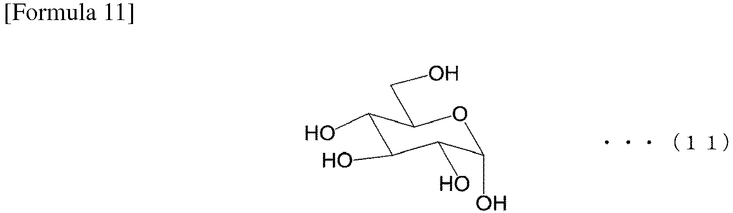

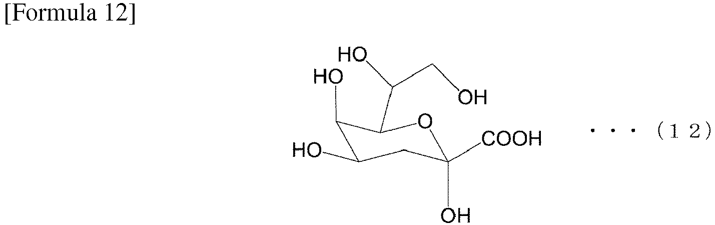

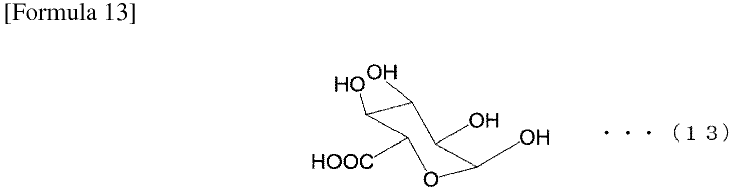

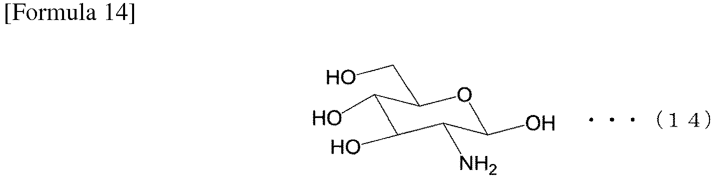

### (5) Summary

In summary of the results (1) to (4), in Formula (A) and Formula (B), it was specified that the purified product contains a compound in which X¹ and X² are hexose and phosphate groups (a compound represented by Formula (A1) and a compound represented by Formula (B1)). In the above-mentioned (2), as shown in Formula (9), it was estimated that a hydroxyl group was bonded to the carbon at the first position of the β-1,6-diglucosamine skeleton. On the other hand, in the above (4), as shown in the schematic diagram of FIG. 21, it was estimated that a phosphate group was attached to the carbon at the 1-position of the β-1,6-diglucosamine skeleton. The latter phosphate group was identified as having the correct structure for the following reasons. That is, in the above-mentioned (2), the purified product is subjected to a predetermined treatment to obtain the decomposition product, and when a weak acid is used in this process, it is generally known that a phosphate group bound to glucosamine on the right side of the two molecules of glucosamine is dropped and the-OH group is replaced with a high frequency.

### (6) NMR analysis of the carbohydrate moiety

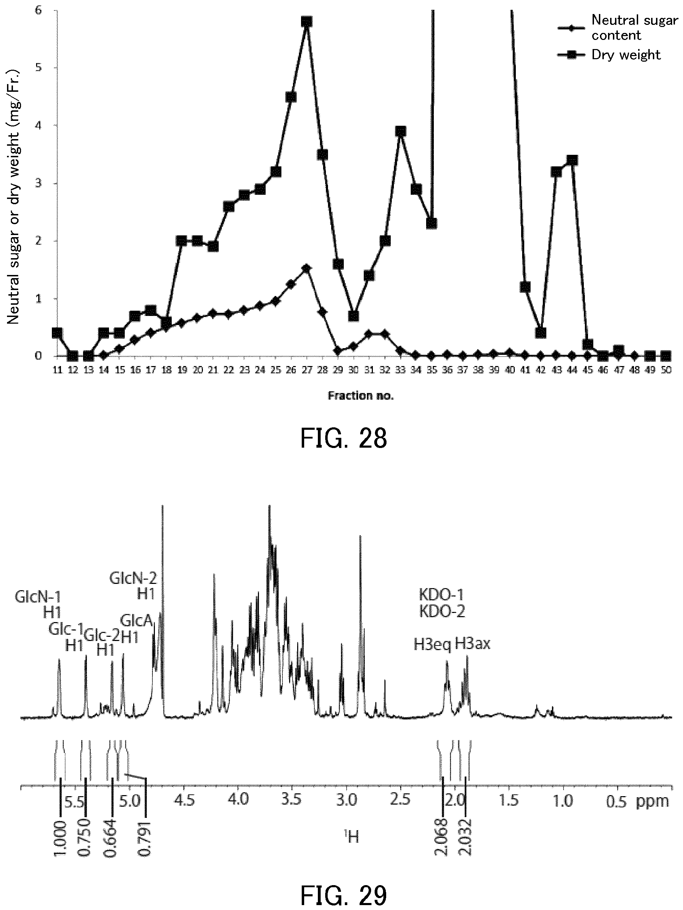

The hydrazine-decomposed product obtained in the above (4) was treated with 4 mol/L KOH, and the liberated carbohydrate moiety was fractionated and purified by gel-filtration column chromatography (Bio-gel P4 media Extra fine <45 µm (wet):#150-4128 manufactured by Bio-Rad). The graph of FIG. 28 shows the gel filtration pattern.

Fractions No. 14-18 in FIG. 28 were mixed (dry weight: 4.8 mg) and the mixture was dissolved in 500 µL of D₂O (99.96% D) to obtain a sample for NMR-measurement (hereinafter referred to as "Sample A"). The NMR measurement was performed under the following conditions. Measurement conditions:
Equipment: DRX500 and ADVANCE600 spectrometer (manufactured by BrukerBioSpin Corporation)
Probes: cryogenic TXI probe, a TXI probe and a BBO probe
Probe temperature: 25°C
Assay: ID ¹H, ID ¹H-selective TOCSY, ¹H-selective NOESY, ¹H-selective ROSEY, ID ¹³C, ID ³¹P, 2D ¹H-¹H DQF-COSY, HOHANA, the NOESY, the ROESY, ¹H-¹³C HSQC-TOCSY, ¹H-¹³C HSQC-NOESY, ¹H-¹³C HMBC, ¹H-³¹P HMBC

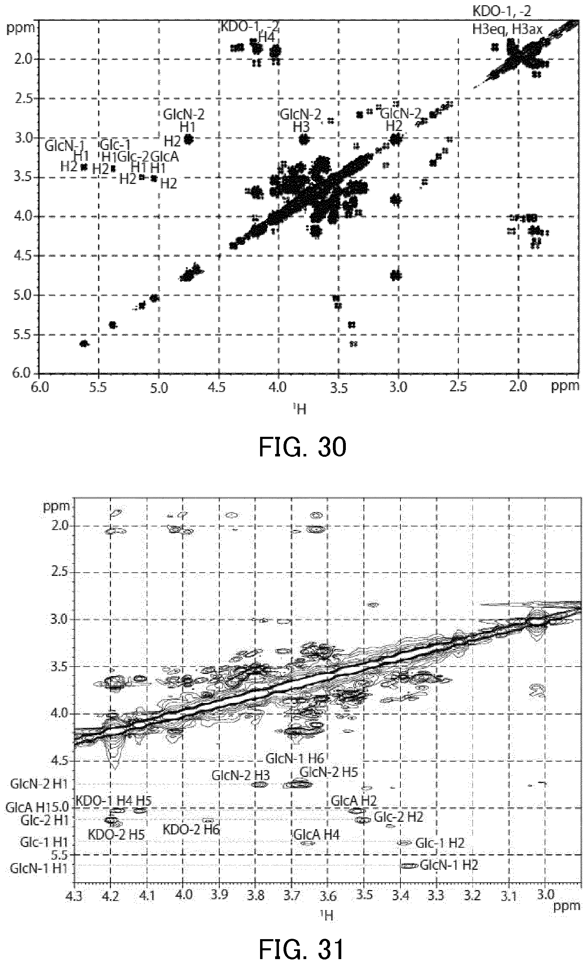

First, signals derived from the anomeric positions of each sugar residue (H1) were identified, and signals within the residue (H2-H6 for glucose and glucosamine residues and H2-H5 for glucuronide residues) were identified from the DQF-COSY, HOHANA, and ¹H-¹³C HSQC-TOCSY spectra based on the anomeric signals. The results are shown in FIGs. 29 and 30. FIG. 29 is a spectrum obtained by subjecting the sample A to ¹HNMR measurement, and FIG. 30 is a spectrum obtained by subjecting the sample A to 2D DQF - COSY measurement. In FIGs. 29 and 30, Glc corresponds to Hex in FIGs. 22 to 26, KDO corresponds to Kdo in FIGs. 22 to 26, GlcA corresponds to HexU in FIGs. 22 to 26, and GlcN corresponds to HexN in FIGs. 22 to 26, and the same is true thereafter. In the case of KDO, the signal (H4-H8) within the KDO residue was assigned based on the proton (H3ax, H3eq) at position 3.

Binding between sugar residues was identified by the NOE signal between the sugar residues observed in the NOESY spectrum (FIG. 31) and the correlated signal between the sugar residues observed in the ¹H-¹³C HMBC spectrum. FIG. 31 is a spectrum obtained by subjecting the sample A to 2D NOESY measurements.

The binding mode (α/β) of the sugar residues was determined by ¹J (Cl, H1) as follows. ¹J (C1, H1)
GlcN-1 174 Hz (α)
GlcN-2 164 Hz (β)
GlcA 171 Hz (α)
Glc-1 173 Hz (α)
Glc-2 171 Hz (α)

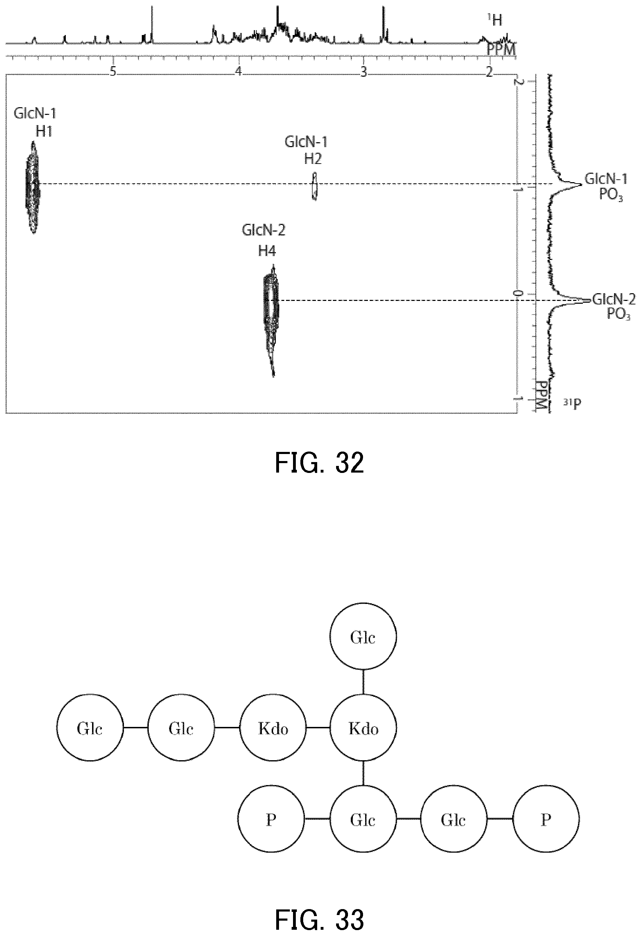

The presence and binding sites of phosphate groups were revealed by ID ³¹P and ¹H-¹³C HMBC. The results are shown in FIG. 32. FIG. 32 is a spectrum obtained by subjecting Sample A to 2D ¹H - ³¹P HMBC measurements. Of the two phosphate groups, one was attached to position 1 of GlcN-1 and the other was attached to position 4 of GlcN-2.

From the above results, the structure of the sample A was specified as shown in the schematic diagram of FIG. 33. From this result, it was specified that the compound before the hydrazine decomposition of the hydrazine decomposition product obtained in the above (4) contains a compound (a compound represented by the Formula (A2) and a compound represented by the Formula (B2)) in which X¹ and X² are both hydroxyl groups in the Formulae (A) and (B). The structural formula of these compounds using the monosaccharide symbol is as follows. FIG. 33 shows a sugar chain structural formula according to a chair conformation.

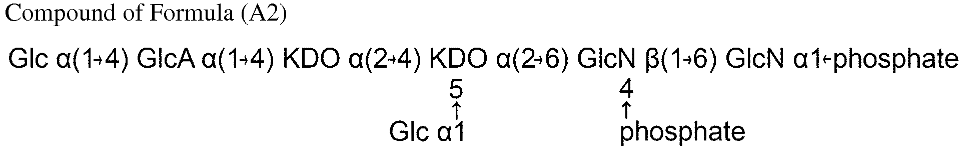

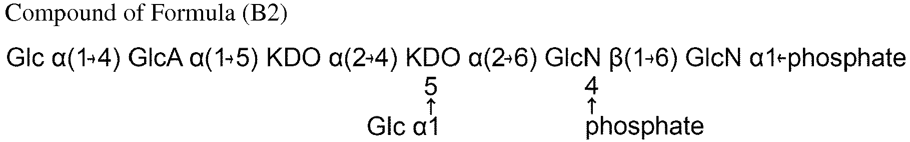

### [Example 3]

It was confirmed that the compound represented by the Formula (A1), the compound represented by the Formula (B1), the compound represented by the Formula (A2), and the compound represented by the Formula (B2) have an anti-inflammatory effect.

### (1) Preparation of test solution

The purified product obtained in Example 1 (the mixture of the compound represented by Formula (A1), the compound represented by Formula (B1), the compound represented by Formula (A2), and the compound represented by Formula (B2)) was dissolved in water for injection to a concentration of 2 mg/mL and stored at 4°C, and the solution was heated at 37°C for 5 minutes and sonicated at 37°C for 1 minute. 10 µL of the sonicated solution was added to 990 µL of the culture solution having the following composition to thoroughly mix, and a solution having a concentration of 20,000 ng/mL was prepared, which was serially diluted using the culture solution to obtain six test solutions having concentrations of 2,000 ng/mL, 200 ng/mL, 20 ng/mL, 2 ng/mL, and 0.2 ng/mL. Since the six test solutions are diluted twice when added to the cells, the final concentrations of the purified products are 10,000 ng/mL, 1,000 ng/mL, 100 ng/mL, 10 ng/mL, 1 ng/mL, and 0.1 ng/mL, respectively.

### Medium composition

DMEM medium 500 ml
Fetal calf serum 55.5 mL
penicillin-streptomycin-glutamine (100x) 5.6 mL

### (2) Addition of test solution

The culture medium was added to Human Embryonic Kidney cells (manufactured by InvivoGen) into which human TLR4 gene had been transfected, and solution were prepared in which the concentration of the cell was 4 × 10⁵ cells/mL. The solution was seeded in 96-well flat-bottomed plates in 100 µL portions, i.e., 4 x 10⁴ cells/100 µL/well. After seeding, the culture was cultured at 37°C in a 5% CO₂ for 24 hours, then the culture supernatant was removed, and 100 µL of the above-described culture medium was added to each well. Next, 100 µL of the test solution was added to each well. After adding the test solution, the culture was incubated at 37°C in a 5% CO₂ for 24 hours.

### (3) Measurement of IL-8 concentration

Culture supernatants from the wells were collected and the concentrations of Interleukin-8 (IL-8) were measured using Human IL-8 ELISA MAX (registered trademark) Standard (Biolegend).

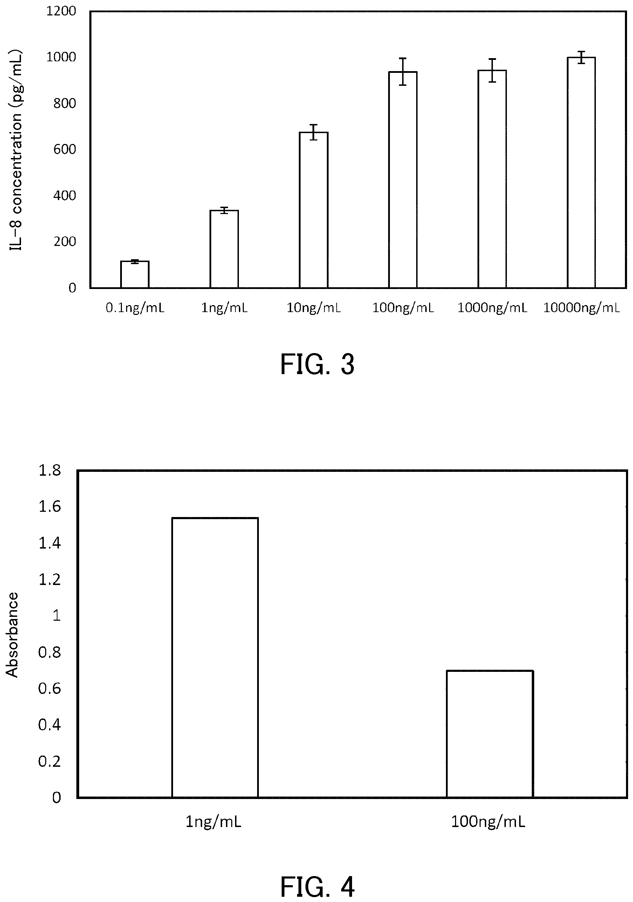

The measurement results are shown in the graph of FIG. 3. In FIG. 3, the vertical axis represents the concentration of IL-8, and the horizontal axis represents the concentration of the test solution. As shown in FIG. 3, the concentration of IL-8 increased depending on the concentration of the test solution. Here, production of IL-8 is generally known to be an indicator of TLR4 activation, and as described above, it has been reported that activation of TLR4 promotes the production of type I interferons having anti-inflammatory effects. Thus, an increase in the concentration of IL-8 implies activation of the anti-inflammatory effect. Therefore, from these results, it was confirmed that the purified product (the mixture of the compound represented by Formula (A1), the compound represented by Formula (B1), the compound represented by Formula (A2), and the compounds represented by Formula (B2)) had an anti-inflammatory effect. Equivalent results were obtained even by using, in place of the purified product, the compound represented by Formula (A1), the compound represented by Formula (B1), the compound represented by Formula (A2), or the compound represented by Formula (B2).

### [Example 4]

It was confirmed that the compound represented by the Formula (A1), the compound represented by the Formula (B1), the compound represented by the Formula (A2), and the compound represented by the Formula (B2) have an anti-inflammatory effect.

### (1) Preparation of test solution

In the same manner as "(1) Preparation of test solution" in Example 3, a solution having a concentration of the purified product of 20,000 ng/mL was prepared, and the solution was serially diluted with a culture solution having the following composition, thereby obtaining two test solutions having 100 ng/mL and 10,000 ng/mL. Since the two test solutions are diluted 100-fold when added to the cells, the final concentrations of the purified product are 1 ng/mL and 100 ng/mL, respectively.

### Medium composition

RPMI1640 medium 500 ml
immobilized fetal bovine serum 55.5 mL
penicillin-streptomycin-glutamine (100x) 5.6 mL

### (2) Addition of test solution

The culture medium was added to mouse macrophage cells (RAW264.7, ATCC) to prepare solutions at concentrations of 1.067 x 10⁶ cells/mL. The solutions were seeded in 6 well plates in 3 mL portions, i.e., 3.2 x 10⁶ cells/3 mL/well. After seeding, the test solution was incubated at 37°C in a 5% CO₂ for 2 hours, and 30 µL of the test solution was added to each well. After adding the test solution, the test solution was incubated at 37°C in a 5% CO₂ for 24 hours. The culture supernatant of each of the wells was then collected in a 15 mL tube. Then, 1 mL of the fresh culture solution was added to each of the wells, and after spreading over the entire well, the operation of collecting the culture solution into the 15 mL tube was repeated twice. Thereafter, the culture solution collected in the 15 mL tube was centrifuged at 1,000 rpm for 3 minutes, the supernatant was removed, and 3 mL of a new culture solution at 37°C was added to suspend the culture solution, and centrifuged again under the same conditions. After centrifugation, the supernatant was removed, and 2 mL of the above-mentioned culture solution at 37°C was added to the precipitate and suspended. Then, 2 mL of the suspension was added to each well to which 1 mL of a new culture medium had been added in advance. Thereafter, 30 µL of a new culture medium was added (hereinafter, this step is referred to as a "culture medium addition step"). After incubation at 37°C in a 5% CO₂ for 30 minutes, the culture supernatants of the wells were collected in 15-mL tubes, centrifuged at 1,000 rpm for 3 minutes, and the supernatants were removed by an aspirator. In addition, to the wells of the 6-well plate from which the culture supernatant was removed, 3 mL of cooled PBS (Phosphate buffered saline)/Phosphatase Inhibitors solution was added, the cells were removed by pipetting, and the cell suspension was added to the 15-mL tubes. The 15 mL tube was centrifuged at 1,000 rpm for 3 minutes, the supernatant was removed by an aspirator, and 0.5 mL of the cooled PBS/Phosphatase Inhibitors solution was added to the 15 mL tube and suspended to prepare a cell suspension. Nucleoproteins were extracted from the cell suspensions using Nuclear Extract kit (manufactured by Active Motif).

### (3) Determination of nuclear NF-KB levels

The amount of NF-KB in the nucleoprotein was confirmed by measuring the absorbance using the Trans AM NF κB p65 kit (manufactured by Active Motif).

The results of the confirmation are shown in the graph of FIG. 4. In FIG. 4, the vertical axis represents the absorbance, and the horizontal axis represents the concentration of the test solution. As shown in FIG. 4, the higher the concentration of the test solution, the lower the absorbance, indicating that the amount of NF-KB in the nucleoprotein decreases as the concentration of the test solution increases. From this result, it was confirmed that the production of NF-KB, which is one type of inflammatory cytokine, was suppressed by the purification product, that is, the purified product (the mixture of the compound represented by Formula (A1), the compound represented by Formula (B1), the compound represented by Formula (A2), and the compound represented by Formula (B2)) had an anti-inflammatory effect. Equivalent results were obtained even by using, in place of the purified product, the compound represented by Formula (A1), the compound represented by Formula (B1), the compound represented by Formula (A2), or the compound represented by Formula (B2).

### [Example 5]

It was confirmed that the compound represented by the Formula (A1), the compound represented by the Formula (B1), the compound represented by the Formula (A2), and the compound represented by the Formula (B2) have an anti-inflammatory effect.

In this example, an experiment was conducted in the same manner as in Example 4 except for the following two points, and the amount of NF-κB in the nucleoprotein was confirmed by measuring the absorbance. That is, in this example, three types of test solutions were used as the test solution, in which the concentration of the purified product was 100 ng/mL, 10,000 ng/mL, and 1,000,000 ng/mL. Since the three test solutions are diluted 100-fold when added to the cells, the final concentrations of the purified products are 1 ng/mL, 100 ng/mL, and 10,000 ng/mL, respectively. In the present embodiment, in the culture medium adding step in Example 3, 30 µL of the culture medium containing LPS (Lipopolysaccharide, Pantoea agglomerans, manufactured by Natural Immune Application Technology Laboratories, Ltd., hereinafter referred to as "LPSp") purified from Pantoea agglomerans, which is a gram-negative bacterium with a final concentration of 100 ng/mL, was added instead of 30 µL of the culture medium.

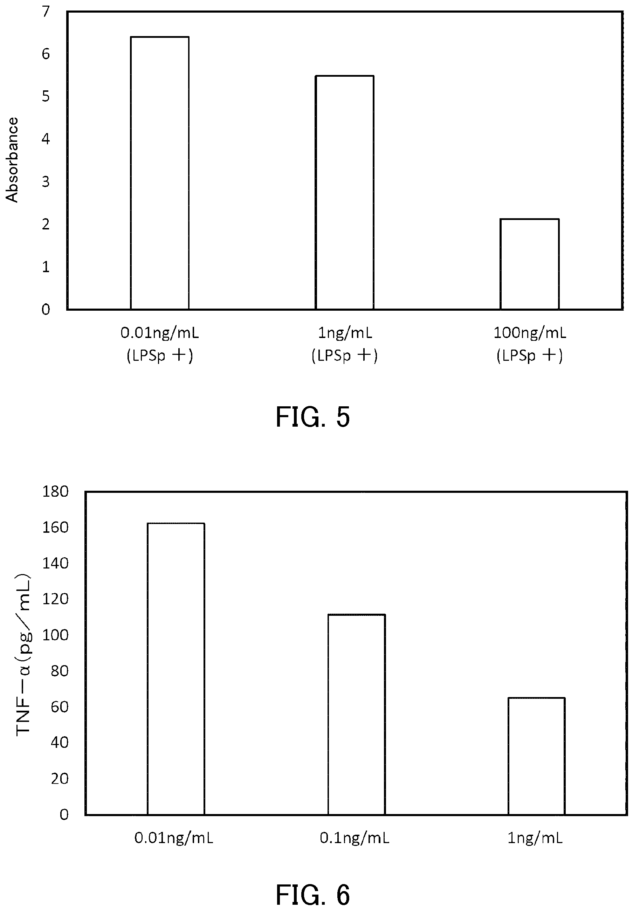

The results of the confirmation are shown in the graph of FIG. 5. In FIG. 5, the vertical axis represents the absorbance, and the horizontal axis represents the concentration of the test solution. As shown in FIG. 5, the higher the concentration of the test solution, the lower the absorbance, indicating that the amount of NF-κB in the nucleoprotein decreases as the concentration of the test solution increases. From this result, it was confirmed that the production of NF-κB, which is one type of inflammatory cytokine, was suppressed by the purification product, that is, the purified product (the mixture of the compound represented by Formula (A1), the compound represented by Formula (B1), the compound represented by Formula (A2), and the compound represented by Formula (B2)) had an anti-inflammatory effect. Equivalent results were obtained even by using, in place of the purified product, the compound represented by Formula (A1), the compound represented by Formula (B1), the compound represented by Formula (A2), or the compound represented by Formula (B2).

### [Example 6]

It was confirmed that the compound represented by the Formula (A1), the compound represented by the Formula (B1), the compound represented by the Formula (A2), and the compound represented by the Formula (B2) have an anti-inflammatory effect.

### (1) Preparation of test solution

In the same manner as "(1) Preparation of test solution" in Example 3, a solution having a concentration of the purified product of 20,000 ng/mL was prepared, and the solution was serially diluted with a culture solution having the following composition, thereby obtaining a total of three test solutions having a concentration of 2 ng/mL, 0.2 ng/mL, and 0.02 ng/mL. Since the three test solutions are diluted twice when added to the cells, the final concentrations of the purified products are 1 ng/mL, 0.1 ng/mL, and 0.01 ng/mL, respectively.

### Medium composition

RPMI1640 medium 500 ml
Fetal calf serum 55.5 mL
kanamycin sulfate 0.11 mL
ampicillin sodium 0.134 mL

### (2) Addition of test solution

The culture medium was added to mouse macrophage cells (RAW264.7, ATCC) to prepare solutions at concentrations of 4 x 10⁵ cells/mL. The solutions were seeded in 96-well flat-bottomed plates in 100 µL portions, i.e., 4 x 10⁴ cells/100 µL/well. After seeding, the cells were incubated at 37°C in 5% CO₂ for 2 hours until they adhered to the bottoms of the wells and extended. Next, 100 µL of the test solution was added to each well. After adding the test solution, the cells were incubated at 37°C in a 5% CO₂ for 24 hours. After the incubation, the culture supernatant of the wells was removed, 150 µl of the above-mentioned culture solution was newly added (hereinafter, this step is referred to as a "culture solution adding step"), and the wells were incubated at 37°C in a 5% CO₂ for 24 hours.

### (3) Measurement of TNF-α concentration

After incubation, 50 µl of culture supernatant from each well was collected, transferred to each well of new 96-well flat-bottom plates, and absorbance was measured using mouse TNF α assay kit (manufactured by Biolegend) and converted to TNF-α concentrations.

The measurement results are shown in the graph of FIG. 6. In FIG. 6, the vertical axis represents the TNF-α concentration, and the horizontal axis represents the concentration of the test solution. As shown in FIG. 6, the higher the concentration of the test solution, the lower the TNF-α concentration, indicating that the production of TNF-α decreases as the concentration of the test solution increases. From this result, it was confirmed that the production of TNF-α, which is one type of inflammatory cytokine, was suppressed by the purification product, that is, the purified product (the mixture of the compound represented by Formula (A1), the compound represented by Formula (B1), the compound represented by Formula (A2), and the compound represented by Formula (B2)) had an anti-inflammatory effect. Equivalent results were obtained even by using, in place of the purified product, the compound represented by Formula (A1), the compound represented by Formula (B1), the compound represented by Formula (A2), or the compound represented by Formula (B2).

### [Example 7]

It was confirmed that the compound represented by the Formula (A1), the compound represented by the Formula (B1), the compound represented by the Formula (A2), and the compound represented by the Formula (B2) have an anti-inflammatory effect.

In this example, an experiment was carried out in the same manner as in Example 6 except for the following two points, and 50 µL of culture supernatant of each well was collected and TNF-α concentration was measured. That is, in this example, a total of five test solutions were used as the test solution, in which the concentration of the purified product was 20 µg/mL, 2 µg/mL, 200 ng/mL, 20 ng/mL, and 2 ng/mL. Since the five test solutions are diluted twice when added to the cells, the final concentrations of the purified products are 10 µg/mL, 1 µg/mL, 100 ng/mL, 10 ng/mL, and 1 ng/mL, respectively. In this example, in the culture medium adding step in Example 5, 150 µL of the culture medium containing LPSp at a final concentration of 100 ng/mL was added instead of 150 µL of the culture medium.

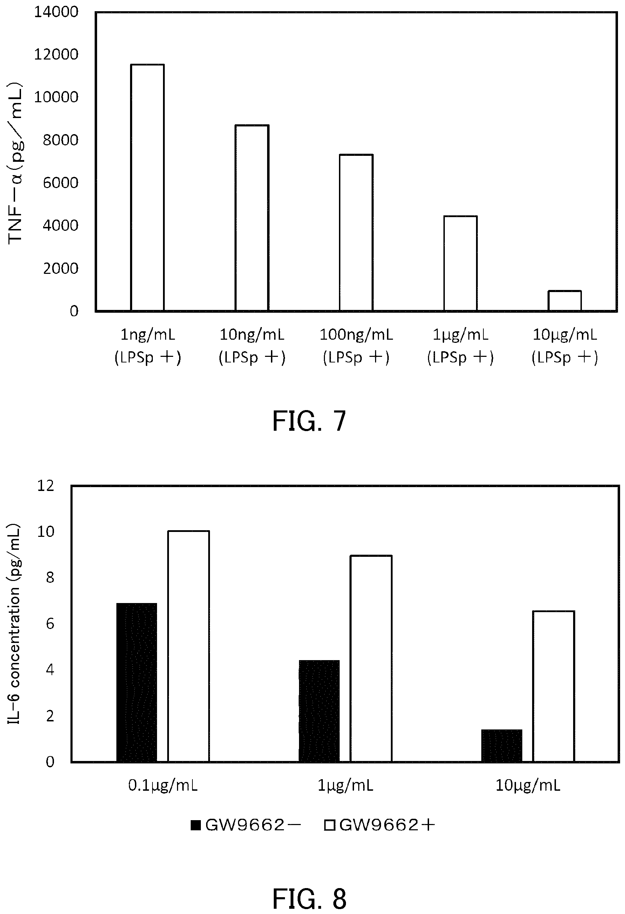

The measurement results are shown in the graph of FIG. 7. In FIG. 7, the vertical axis represents the TNF-α concentration, and the horizontal axis represents the concentration of the test solution. As shown in FIG. 7, the TNF-α concentration became lower as the concentration of the test solution was higher, indicating that the production of TNF-α decreased as the concentration of the test solution was higher. From this result, it was confirmed that the production of TNF-α, which is one type of inflammatory cytokine, was suppressed by the purification product, that is, the purified product (the mixture of the compound represented by Formula (A1), the compound represented by Formula (B1), the compound represented by Formula (A2), and the compound represented by Formula (B2)) had an anti-inflammatory effect. Equivalent results were obtained even by using, in place of the purified product, the compound represented by Formula (A1), the compound represented by Formula (B1), the compound represented by Formula (A2), or the compound represented by Formula (B2).

### [Example 8]

### [Example 8-1]

It was confirmed that the compound represented by the Formula (A1), the compound represented by the Formula (B1), the compound represented by the Formula (A2), and the compound represented by the Formula (B2) have an anti-inflammatory effect.

### (1) Preparation of test solution

In the same manner as "(1) Preparation of test solution" in Example 3, a solution having a concentration of the purified product of 20,000 ng/mL was prepared, and the solution was serially diluted with a culture solution having the following composition, thereby obtaining a total of three test solutions having a concentration of 400 ng/mL, 4,000 ng/mL, and 40000 ng/mL. Since the three test solutions are diluted four-fold when added to the cells, the final concentrations of the purified products are 100 ng/mL, 1,000 ng/mL, and 10,000 ng/mL, respectively.

### Medium composition

RPMI1640 medium 500 ml
immobilized fetal bovine serum 55.5 mL
penicillin-streptomycin-glutamine (100x) 5.6 mL

### (2) Addition of test solution

The culture medium was added to cells derived from human peripheral blood monocytes (THP-1, manufactured by DS Pharma Biomedical Co., Ltd.) to prepare solutions in which the concentrations of the cells were 4 x 10⁵ cells/mL. The solutions were seeded in 24 well plates in 500 µL portions, i.e., 2.0 x 10⁵ cells/500 µL/well. After seeding, 250 µL of the culture medium or 250 µL of the culture medium containing 40 µmol/L of GW9662 (manufactured by Wako Pure Chemical Industries, Ltd.) was added to the wells. The GW9662 is an inhibitor of PPARγ(Peroxisome proliferator-activated receptor γ) which is one type of nuclear receptor. After the addition, the mixture was incubated at 37°C in a 5% CO₂ for 1 hour. Thereafter, 250 µl of the test solution was added to each well, and the wells were incubated at 37°C in a 5% CO₂ for 22 hours. After culturing, the culture supernatant of each well was collected in a 2 mL tube. A new 0.5 mL of the culture medium was added to each well, spread over the whole well, and collected in the 2 mL tube. To each empty well was added 0.49 mL of fresh culture medium. The culture solution collected in the 2 mL tube was centrifuged at 1,000 rpm for 5 minutes, the supernatant was removed, and 1 mL of the fresh culture solution was added to suspend, and centrifuged again under the same conditions. After centrifugation, the supernatant was removed and 0.49 mL of culture medium in each well was added to the 2 mL tube, suspended and returned to each well. Then, 250 µL of the culture medium or 250 µL of the culture medium containing 40 µmol/L of the above GW9662 was added to the wells, and the wells were cultured at 37°C in a 5% CO₂ for 1 hour. After the incubation, 250 µL of the culture medium containing LPSp was added to the wells so that the final concentration of the LPSp was 100 ng/mL, and the wells were incubated at 37°C in a 5% CO₂ for 22 hours.

### (3) Measurement of IL-8 concentration

After culturing, the culture supernatants of the wells were collected in tubes, centrifuged, and the supernatants were collected in 1.5-mL tubes. The absorbance was measured using Human IL-6 ELISA MAX Deluxe (Biolegend Corporation), and the concentrations of Interleukin-6 (IL-6) were calculated.

The measurement results are shown in the graph of FIG. 8. In FIG. 8, the vertical axis represents the concentration of IL-6, and the horizontal axis represents the concentration of the test solution. As shown in FIG. 8, in case of GW9662 -, i.e., when PPARγ was not inhibited, IL-6 concentration was lower as the concentration of the test solution was higher, indicating that IL-6 production was lower as the concentration of the test solution was higher. On the other hand, in case of GW9662 +, i.e., when PPARγ was inhibited, IL-6 levels decreased with increasing concentrations of the test solution, indicating that IL-6 production decreased with increasing concentrations of the test solution, but the degree of reduction was not as pronounced as when PPARγ was not inhibited. From this result, it was confirmed that the production of IL-6, which is one type of inflammatory cytokine, was suppressed by the purification product, that is, the purified product (the mixture of the compound represented by Formula (A1), the compound represented by Formula (B1), the compound represented by Formula (A2), and the compound represented by Formula (B2)) had an anti-inflammatory effect. It was also suggested that PPARγ is involved in suppressing the production of IL-6 by the purified product (the mixture of the compound represented by Formula (A1), the compound represented by Formula (B1), the compound represented by Formula (A2), and the compound represented by Formula (B2)). Equivalent results were obtained even by using, in place of the purified product, the compound represented by Formula (A1), the compound represented by Formula (B1), the compound represented by Formula (A2), or the compound represented by Formula (B2).

### [Example 8-2]

The fact that the compound represented by the Formula (A1), the compound represented by the Formula (B1), the compound represented by the Formula (A2), and the compound represented by the Formula (B2) promote the expression of the PPARγ gene was confirmed by the CAGE(Cap Analysis of Gene Expression) method developed by Institute of Physical and Chemical Research.

The same culture medium as in Example 8-1(2) was added to the THP-1 cells to prepare solutions in which the concentrations of the cells were 5 x 10⁵ cells/mL. The expression level of the PPARγ gene in this solution was measured by the CAGE method and found to be 3.97 TPM (Tags Per Million). After the measurement, the cell solution was divided into the following four groups. That is, there are four groups: a group in which a solution of the purified product is added to the cell solution so as to have a final concentration of 1 µg/mL (Example 8-2A), a group in which a solution of the purified product is added to the cell solution so as to have a final concentration of 10 µg/mL (Example 8-2B), a group in which nothing is added to the cell solution (Comparative Example 8-2A), and a group in which a LPSp solution is added to the cell solution so as to have a final concentration of 100 ng/mL (Comparative Example 8-2B). After culturing the four groups for 3 hours, the expression level of the PPARγ gene was measured by the CAGE method. The results are shown in FIG. 27. FIG. 27 is a graph showing the expression levels of the PPARγ genes. In FIG. 27, the horizontal axis represents the incubation time, and the vertical axis represents the expression amount (TPM) of the PPARγ gene. As shown in FIG. 27, in Example 8-2A, the expression level of the PPARγ gene increased drastically from 3.97 TPM to 5.86 TPM during 3 hours, and in Example 8-2B, the expression level of the PPARγ gene also increased drastically from 3.97 TPM to 5.90 TPM during 3 hours. In contrast, in Comparative Example 8-2A, the expression level of the PPARγ gene decreased slightly from 3.97 TPM to 3.89 TPM during 3 hours, and in Comparative Example 8-2B, the expression level of the PPARγ gene increased from 3.97 TPM to 4.31 TPM during 3 hours, but the degree of the increase was not significant. From this result, it was confirmed that the purified product (the mixture of the compound represented by Formula (A1), the compound represented by Formula (B1), the compound represented by Formula (A2), and the compound represented by Formula (B2)) promotes expression of the PPARγ gene. Equivalent results were obtained even by using, in place of the purified product, the compound represented by Formula (A1), the compound represented by Formula (B1), the compound represented by Formula (A2), or the compound represented by Formula (B2).

### [Example 9]

It was confirmed that the compound represented by the Formula (A1), the compound represented by the Formula (B1), the compound represented by the Formula (A2), and the compound represented by the Formula (B2) have an anticancer effect on lung cancer.

### (1) Preparation of cancer-bearing mice

Suspensions of Lewis lung cancer cell lines (Lewis lung carcinoma: 3LL, obtained from JCRB cell banks) were subcutaneously administered to the ventral region of five male C57 BL/6J mice aged 6 weeks so that the cancer cell dose per animal was 2.5 x 10⁵ cells per 200 µL. Two weeks after dosing, tumors were excised from one of the mice and washed on dishes with PBS (-). Next, the washed tumor was made into a finely divided tumor fragment of about 2 mm square, the tumor fragment was transferred to a 50 mL tube, 5 mL of collagenase solution was added, and the mixture was warmed at 37°C for 10 minutes. Thereafter, the collagenase solution containing the tumor fragments was pipetted into finer tumor fragments, and the collagenase solution containing the tumor fragments was transferred to another 50 mL tube and cooled on ice. After cooling, an additional 5 mL of collagenase solution was added to the collagenase solution containing the tumor fragments, and the same procedure was repeated 5 times until no tumor fragments could be observed. Thereafter, the collagenase solution containing the tumor fragment was filtered through a cell strainer (mesh size: 70 µm, manufactured by BD Corporation), and the filtrate was centrifuged at 1200 rpm for 7 minutes. After centrifugation, the supernatant was removed, 20 ml of RPMI1260 medium (serum-free) was added to the precipitate, and after suspension by inversion mixing, the precipitate was centrifuged at 1200 rpm for 7 minutes. After centrifugation, the supernatant was removed, and the precipitate was washed twice with PBS (-), and then 10 mL of PBS (-) was added and suspended to prepare a cancer cell suspension. The cancer cell suspension was subcutaneously administered to the ventral portion of the 12 mice so that the cancer cell dose per animal was 2.5 x 10⁵ cells per 200 µL. Fourteen days after dosing, tumors were excised from 10 of the mice and cancer cell suspensions were prepared in the same manner as described above. The cancer cell suspensions were administered intracutaneously into the abdomen of the 108 mice so that the cancer cell dose per animal was 2.5 x 10⁵ cells per 50 µL. After administration, mice were divided into six groups (n = 6 in each group) as shown below at the time point when the tumor size of each mouse became about 5 mm in diameter (day 8 after administration).

**[Table 5]**

| | Administration sample | Administration of CY |
|---|---|---|
| Group 1 (Example 9-1) | Purification product (ip) obtained in Example 1 | |
| Group 2 (Example 9-2) | Purification product (ip) obtained in Example 1 | + |
| Group 3 (Example 9-3) | Purification product (po) obtained in Example 1 | |
| Group 4 (Example 9-4) | Purification product (po) obtained in Example 1 | + |
| Group 5 (Comparative Example 9-1) | Saline solution (ip) | - |
| Group 6 (Reference Example 9-1) | - | + |

| | | |
|---|---|---|
| ip = intraperitoneal administration po = free intake CY = cyclophosphamide (anti-cancer agent) | | |

### (2) Drug administration

After grouping, each group was dosed with the substances listed in Table 5 above. The administration of the purification product was performed once immediately after grouping so that the intake of the purified product in the mice was 0.5 mg/10 mL/kg in the intraperitoneal administration (ip) of Example 9-1 and Example 9-2, and was started immediately after grouping using a water supply bottle containing a solution of the purified product having a concentration of 1 µg/mL in the case of free intake (po) of Example 9-3 and Example 9-4. The water supply bottle was changed to a new one every three days. The saline solution of Comparative Example 9-1 was administered once immediately after grouping so that the saline intake of mice was 10 mL/kg by ip. The administration of CY (cyclophosphamide, an anti-cancer agent against lung cancer, Wako Pure Chemical Industries, Ltd.) in Examples 9-2, 9-4, and Reference Example 9-1 was performed once immediately after grouping so that the CY intake of mice was 100 mg/10 mL/kg by ip.

### (3) Measurement of tumor volume

The major and minor diameters of the tumors were measured using calipers on Days 3, 6 and 9 on the day of grouping as day 0, and the tumor volume was calculated based on these measurements. Tumor volume calculations were performed according to the methods described in Shime et al. (Shime H, et al, "Toll-like receptor 3 signaling converts tumor-supporting myeloid cells to tumoricidal effectors", Proc Natl Acad Sci USA, February 7, 2012, Vol. 109, no. 6, p. 2066-2071) using the formula: major axis (mm) x minor axis (mm)² x 0.4 = tumor volume (mm3).

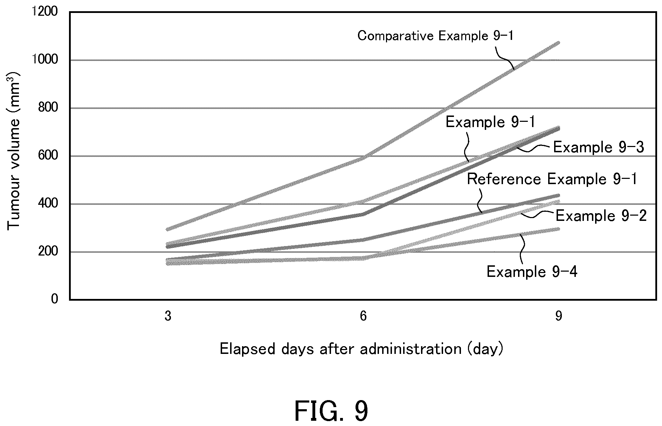

The measurement results are shown in the graph of FIG. 9. In FIG. 9, the vertical axis represents the tumour volume (mm³), and the horizontal axis represents the number of days elapsed after grouping when the day of grouping is day 0. As shown in FIG. 9, in Example 9-1 and Example 9-2 in which the purified product was administered, the tumor volume was smaller than that in Comparative Example 9-1 in which physiological saline was administered. From this, it was confirmed that the purified product (the mixture of the compound represented by Formula (A1), the compound represented by Formula (B1), the compound represented by Formula (A2), and the compound represented by Formula (B2)) has an anticancer effect on lung cancer. Further, in Examples 9-2 and 9-4 in which CY was administered in addition to the purified product, the tumor volume was smaller than that in Reference Example 9-1 in which CY alone was administered. This confirms that the purified product (the mixture of the compound represented by Formula (A1), the compound represented by Formula (B1), the compound represented by Formula (A2), and the compound represented by Formula (B2)) has an effect of enhancing the anticancer effect of CY on lung cancer. In place of the purified product, the compound represented by Formula (A1), the compound represented by Formula (B1), the compound represented by Formula (A2), or the compound represented by Formula (B2) can be used to obtain equivalent results.

While the present invention has been described above with reference to illustrative embodiments and examples, the present invention is by no means limited thereto. Various changes and variations that may become apparent to those skilled in the art may be made in the configuration and specifics of the present invention without departing from the scope of the present invention.

This application claims priority from Japanese Patent Application No. 2016-186116 filed on September 23, 2016 and Japanese Patent Application No. 2016-238863 filed on December 8, 2016. The entire subject matters of the Japanese Patent Applications are incorporated herein by reference.

### Industrial Applicability

As described above, the compound of the present invention or its salt can be used as an anti-inflammatory agent, an anti-cancer agent against lung cancer, or the like. The compound or the salt thereof of the present invention can be administered over a long period of time because of their high safety.

## Claims

1. A compound represented by Formula (A) or Formula (B) or a salt thereof: 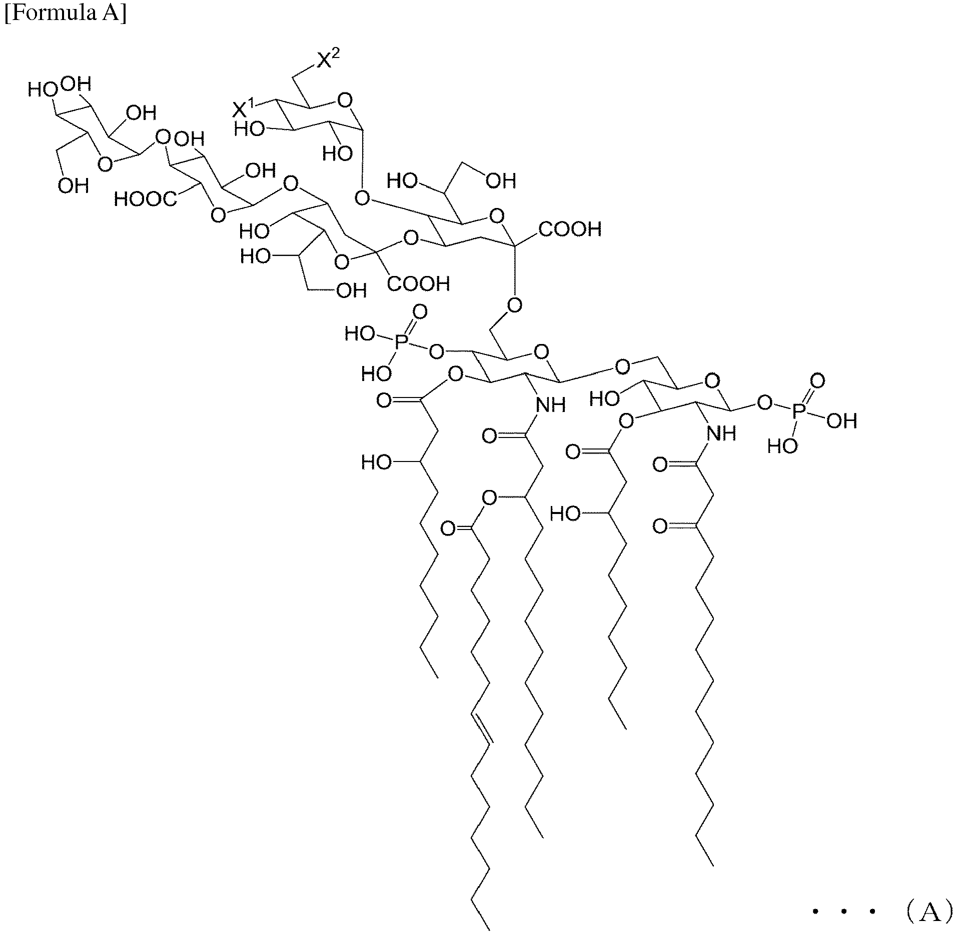 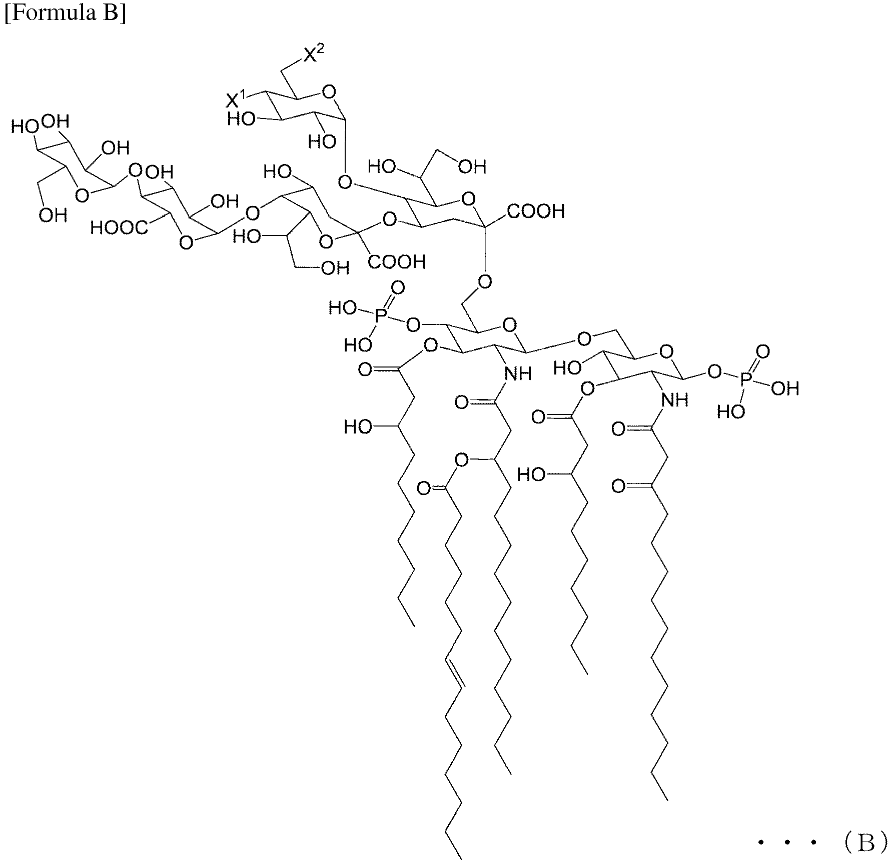 where:
X¹ is a hexose or a hydroxyl group, and
X² is a phosphate group or a hydroxyl group.

2. An anti-inflammatory agent comprising:
the compound or the salt thereof according to claim 1.

3. An anti-cancer agent lung cancer, comprising:
the compound or the salt thereof according to claim 1.

4. A method of producing a compound represented by Formula (A) or Formula (B) or a salt thereof, comprising the steps of:
extracting a crude extract comprising a compound represented by Formula (A) or Formula (B) or a salt thereof from at least one of Rhodobacter azotoformans BP0899 strain (Accession No.: NITE BP-644) or a culture thereof; and
isolating the compound represented by Formula (A) or Formula (B) or a salt thereof from the crude extract to purify: 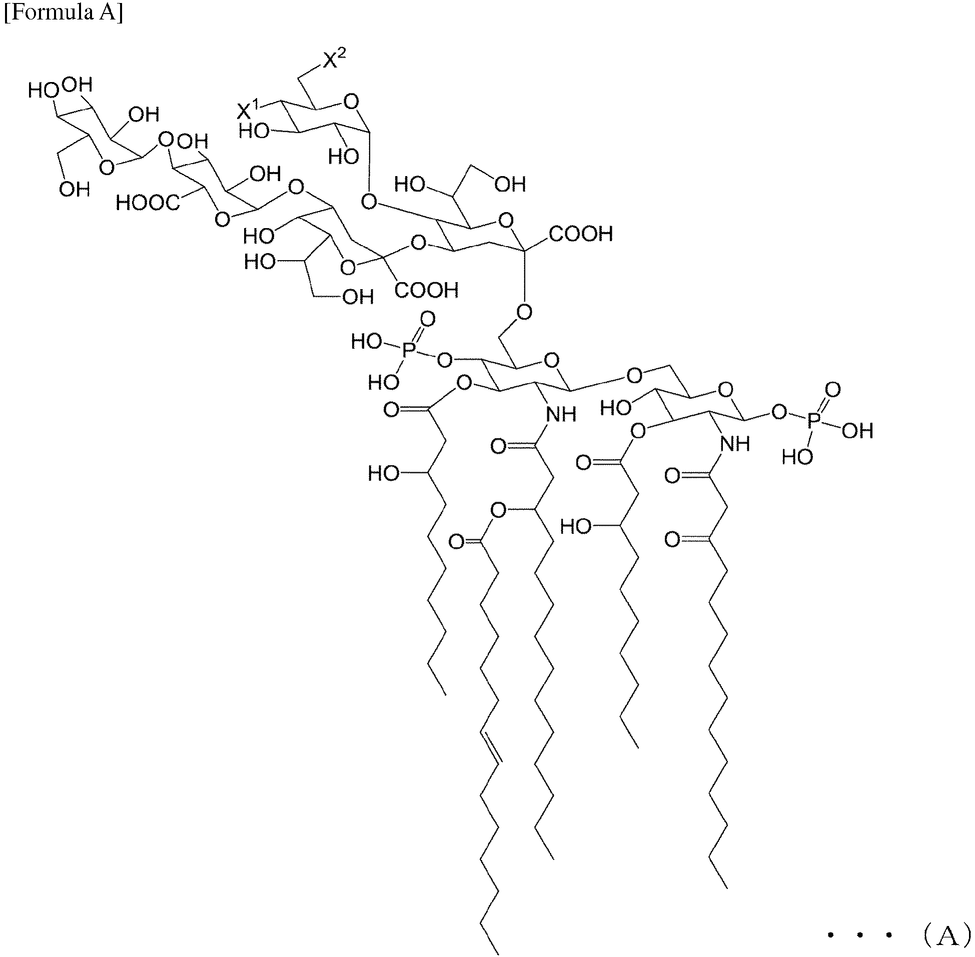 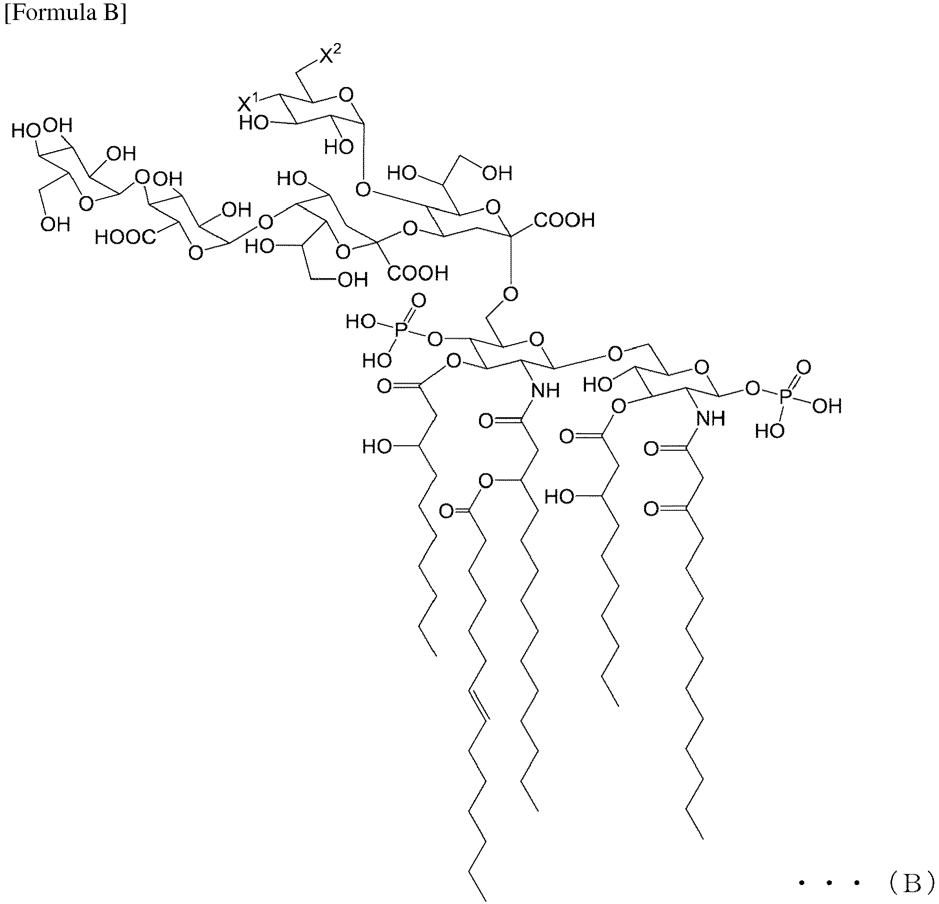 where:
X¹ is a hexose or hydroxyl group, and
X² is a phosphate group or a hydroxyl group.

5. The method according to claim 4, wherein
an extraction process in the crude extraction step is an extraction process using an organic solvent that insolubilizes a protein.

6. The method according to claim 5, wherein the organic solvent is phenol.

7. The method according to any one of claims 4 to 6, wherein
in the crude extraction step, prior to the extraction process, at least one of Rhodobacter azotoformans BP0899 strain (Accession No.: NITE BP 644) or a culture thereof is subjected to a decolorization process of a pigment.

8. The method according to claim 7, wherein
the decolorization process of the pigment is a decolorization process using at least one selected from the group consisting of acetone, methanol, and chloroform.

9. The method according to any one of claims 4 to 8, wherein
in the crude extraction step, the crude extract is subjected to a filtration process.

10. The method according to any one of claims 4 to 9, wherein
in the purification step, the crude extract is subjected to an enzyme treatment process and an extraction process using an organic solvent that insolubilizes a protein.

11. The method according to claim 10, wherein
the enzyme treatment process is an enzyme treatment process using at least one of a nuclease or a protease.

12. The method according to claim 10 or 11, wherein
the organic solvent is phenol.

13. The method according to any one of claims 10 to 12, wherein
in the purification step, an extract after the extraction process is subjected to a filtration process.

14. A method of treating inflammatory diseases, comprising the step of:
administering an anti-inflammatory agent comprising the compound or the salt thereof according to claim 1.

15. A method of treating lung cancer, comprising the step of:
administering an anti-cancer agent lung cancer comprising the compound or the salt thereof according to claim 1.


SEQUENCE LISTING

<110>  TFK Co.,Ltd.

<120>  Compounds or salts thereof, anti-inflammatory drug, anticancer drug
for lung cancer, method for manufacturing the compounds or salts thereof,
method for treating inflammatory diseases and method for treating lung cancer

<130>  TF16007WO

<150>  JP 2016-186116
<151>  2016-09-23

<150>  JP 2016-238863
<151>  2016-12-08

<160>  9

<170>  PatentIn version 3.1

<210>  1
<211>  1427
<212>  DNA
<213>  Rhodobacter azotoformans

<400>  1
gagtttgatc ctggctcaga atgaacgctg gcggcaggcc taacacatgc aagtcgagcg      60

aagtcttcgg acttagcggc ggacgggtga gtaacgcgtg ggaacatgcc caaaggtacg     120

gaatagcccc gggaaactgg gagtaatacc gtatgtgccc ttcgggggaa agatttatcg     180

cctttggatt ggcccgcgtt ggattaggta gttggtgggg taatggccta ccaagccgac     240

gatccatagc tggtttgaga ggatgatcag ccacactggg actgagacac ggcccagact     300

cctacgggag gcagcagtgg ggaatcttag acaatgggcg caagcctgat ctagccatgc     360

cgcgtgatcg atgaaggcct tagggttgta aagatctttc aggtgggaag ataatgacgg     420

taccaccaga agaagccccg gctaactccg tgccagcagc cgcggtaata cggagggggc     480

tagcgttatt cggaattact gggcgtaaag cgcacgtagg cggactggaa agtcaggggt     540

gaaatcccgg ggctcaaccc cggaactgcc tttgaaactc ccagtcttga ggtcgagaga     600

ggtgagtgga attccgagtg tagaggtgaa attcgtagat attcggagga acaccagtgg     660

cgaaggcggc tcactggctc gatactgacg ctgaggtgcg aaagcgtggg gagcaaacag     720

gattagatac cctggtagtc cacgccgtaa acgatgaatg ccagtcgtcg ggcagcatgc     780

tgttcggtga cacacctaac ggattaagca ttccgcctgg ggagtacggc cgcaaggtta     840

aaactcaaag gaattgacgg gggcccgcac aagcggtgga gcatgtggtt taattcgaag     900

caacgcgcag aaccttacca acccttgaca tggcgatcgc ggttccagag atggttcctt     960

cagttcggct ggatcgcaca caggtgctgc atggctgtcg tcagctcgtg tcgtgagatg    1020

ttcggttaag tccggcaacg agcgcaaccc acgtcctcag ttgccagcat tcagttgggc    1080

actctgggga aactgccggt gataagccgg aggaaggtgt ggatgacgtc aagtcctcat    1140

ggcccttacg ggttgggcta cacacgtgct acaatggcag tgacaatggg ttaatcccaa 1200

aaagctgtct cagttcggat tggggtctgc aactcgaccc catgaagtcg gaatcgctag 1260

taatcgcgta acagcatgac gcggtgaata cgttcccggg ccttgtacac accgcccgtc 1320

acaccatggg aattggttct acccgaaggc ggtgcgccaa cctcgcaaga ggaggcagcc 1380

gaccacggta ggatcagtga ctggggtgaa gtcgtaacaa ggtagcc 1427

<210> 2
<211> 19
<212> DNA
<213> Artificial

<220>
<223> a primer

<400> 2
gagtttgatc ctggctcag 19

<210> 3
<211> 19
<212> DNA
<213> Artificial

<220>
<223> a primer

<400> 3
ctcctacggg aggcagcag 19

<210> 4
<211> 21
<212> DNA
<213> Artificial

<220>
<223> a primer

<400> 4
ggattagata ccctggtagt c 21

<210> 5
<211> 16
<212> DNA
<213> Artificial

<220>
<223> a primer

<400> 5
gcaacgagcg caaccc 16

<210> 6
<211> 18
<212> DNA

<213>  Artificial

<220>
<223>  a primer

<400>  6
gtattaccgc ggctgctg                                                    18


<210>  7
<211>  18
<212>  DNA
<213>  Artificial

<220>
<223>  a primer

<400>  7
taccagggta tctaatcc                                                    18


<210>  8
<211>  16
<212>  DNA
<213>  Artificial

<220>
<223>  a primer

<400>  8
ccattgtagc acgtgt                                                      16


<210>  9
<211>  17
<212>  DNA
<213>  Artificial

<220>
<223>  a primer

<400>  9
aaggaggtga tccagcc                                                     17